# EUROPEAN PATENT APPLICATION

(11) **EP 1 837 398 A1**
(43) Date of publication of application: **26.09.2007**
(21) Application number: 05842245.2
(22) Date of filing: 26.12.2005
(51) Int. Cl.: C12N 15/09, G01N 33/68, C12Q 1/02, C12Q 1/68, A01K 67/027, A61K 31/7088, A61K 45/00, A61K 48/00, A61P 1/16, C12N 15/10, G01N 33/15

(54) **DRUGS FOR DISEASES ACCOMPANYING CHANGES IN TOTAL BILE ACID POOL OR LIPID METABOLISM DISORDERS AND METHOD OF SCREENING THESE DRUGS**

(30) Priority: 27.12.2004 JP 2004377833; 21.10.2005 JP 2005307538
(71) Applicant: BANYU PHARMACEUTICAL CO., LTD., Tokyo 102-8667 (JP)
(72) Inventor: MARUYAMA, Takaharu, Tsukuba-shi, Ibaraki 300-2611 (JP); TANAKA, Kenichi, Tsukuba-shi, Ibaraki 300-2611 (JP); TAMAI, Yoshitaka, Tsukuba-shi, Ibaraki 300-2611 (JP); SUZUKI, Jun, Tsukuba-shi, Ibaraki 300-2611 (JP)
(74) Representative: Horgan, James Michael Frederic
(86) International application number: PCT/JP2005/023734
(87) International publication number: WO 2006/070718

(57) **Abstract**

A Gpbar1-deficient mouse is constructed and it is examined whether or not Gpbar1 participates in the regulation of bile acid homeostasis and lipid metabolism. As a result, the total bile acid pool is decreased in the Gpbar1-deficient mouse without showing any change in the fecal bile acid level. A female Gpbar1-deficient mouse having been fed with a high fat feed shows a significant increase in body weight compared with a wild type mouse, which is caused by an increase in fat. These facts suggest that Gpbar1 contributes to the regulation of bile acid homeostasis and lipid metabolism.

## Description

### TECHNICAL FIELD

The present invention relates to drugs for diseases accompanying changes in total bile acid pool or for lipid metabolism disorders, and to a method of screening these drugs. The invention also relates to a test method and a test reagent for diseases accompanying changes in total bile acid pool or for lipid metabolism disorders. Further, the invention relates to a genetically-modified non-human mammal in which the expression of a Gpbar1 gene is artificially inhibited.

### BACKGROUND ART

Bile acid is produced from cholesterol in liver, and it has an extremely important role not only in solubilization of fat in foods but also in maintenance of homeostasis of bile acid and cholesterol (Non-Patent References 1, 2). It is well known that bile acid regulates many biosynthetic enzymes and transporters via activation of farnesoid X receptor (FXR) (Non-Patent References 3, 4). For example, cholesterol 7α-hydrogenase (CYP7A), Na⁺-taurocholate cotransporting polypeptide (NTCP) and bile salt excretory pump (BSEP), which are rate-limiting enzymes in bile acid synthesis, and their transporters are extremely important for homeostasis of bile acid (Non-Patent References 5 to 9).

It is well studied that steroid hormone regulates various genes through classic genome response via stimulation of its nuclear receptor (Non-Patent References 10, 11). However, there exist substantial evidence indicating that some steroid hormones stimulate a secondary messenger owing to rapid non-genomic response (Non-Patent Reference 12). Zhu et al. have identified a membrane progestin receptor (mPR) and clarified that this has a seven-transmembrane domain that is a typical structure of G-protein-coupled receptor (GPCR) (Non-Patent References 13, 14). In cells expressing mPR therein, progestin inhibits cAMP formation, and since the reaction is sensitive to pertussis toxin, it is suggested that mPR is coupled with Gi/o protein. Similarly, bile acid activates nuclear receptors such as FXR, and some data suggest the presence of a bile acid-specific receptor that rapidly stimulates cAMP formation (Non-Patent References 15, 16).

Non-Patent Reference 1: Russell, D. W., and Setchell, K. D. 1992. Bile acid biosynthesis. Biochemistry 31: 4737-4749.
Non-Patent Reference 2: Dietschy, J. M. 1968. Mechanisms for the intestinal absorption of bile acids. J. Lipid Res. 9: 297-309.
Non-Patent Reference 3: Russell, D. W. 2003. The enzymes, regulation, and genetics of bile acid synthesis. Annu. Rev. Biochem. 72: 137-174.
Non-Patent Reference 4: Redinger, R. N. 2003. Nuclear receptors in cholesterol catabolism: molecular biology of the enterohepatic circulation of bile salts and its role in cholesterol homeostasis. J. Lab. Clin. Med. 142: 7-20.
Non-Patent Reference 5: Sinal, C. J., Tohkin, M., Miyata, M., Ward, J. M., Lambert, G., and Gonzalez, F. J. 2000. Targeted disruption of the nuclear receptor FXR/BAR impairs bile acid and lipid homeostasis. Cell 102: 731-744.
Non-Patent Reference 6: Tu, H., Okamoto, A. Y., and Shan, B. 2000. FXR, a bile acid receptor and biological sensor. Trends. Cardiovasc. Med. 10: 30-35.
Non-Patent Reference 7: Chiang, J. Y. L., kimmel, R., Weinberger, C., and Stroup, D. 2000. Famesoid X receptor responds to bile acids and represses cholesterol 7alpha-hydroxylase gene (CYP7A1) transcription. J. Biol. Chem. 275: 10918-10924.
Non-Patent Reference 8: Ananthanarayanan, M., Balasubramanian, N., Makishima, M., Mangelsdorf, D. J., and Suchy, F., J. 2001. Human bile salt export pump promoter is transactivated by the farnesoid X receptor/bile acid receptor. J. Biol. Chem. 276: 28857-28865.
Non-Patent Reference 9: Grober, J., Zaghini, I., Fujii, H., Jones, S. A., Kliewer, S. A., Willson, T. M., Ono, T., and Besnard, P. 1999. Identification of a bile acid-responsive element in the human ileal bile acid-binding protein gene. J. Biol. Chem. 274: 29749-29754.
Non-Patent Reference 10: Beato, M. 1989. Gene regulation by steroid hormones. Cell 56: 335-344.
Non-Patent Reference 11: Aranda, A., and Pascual, A. 2001. Nuclear hormone receptors and gene expression. Physiol. Rev. 81: 1269-1304.
Non-Patent Reference 12: Norman, A. W., Mizwicki, M. T., and Norman, D. P. 2004. Steroid-hormone rapid actions, membrane receptors and a conformational ensemble model. Nat. Rev. Drug Discov. 3: 27-41.
Non-Patent Reference 13: Zhu, Y., Rice, C. D., Pang, Y., Pace, M., and Thomas, P. 2003. Cloning, expression, and characterization of a membrane progestin receptor and evidence it is an intermediary in meiotic maturation of fish oocytes. Proc. Natl. Acad. Sci. USA. 100: 2231-2236.
Non-Patent Reference 14: Zhu, Y., Bond, J., and Thomas, P. 2003. Identification, classification, and partial characterization of genes in humans and other vertebrates homologous to a fish membrane progestin receptor. Proc. Natl. Acad. Sci. U S A. 100: 2237-2242.
Non-Patent Reference 15: Conley, D. R., Coyne, M. J., Bonorris, G. G., Chung, A., and Schoenfield, L. J. 1976. Bile acid stimulation of colonic adenylate cyclase and secretion in the rabbit. Am. J. Dig. Dis. 21: 453-458.
Non-Patent Reference 16: Potter, G. D., Sellin, J. H., and Burlingame, S. M. 1991. Bile acid stimulation of cyclic AMP and ion transport in developing rabbit colon. J. Pediatr. Gastroenterol. Nutr. 13: 335-341.
Non-Patent Reference 17: Maruyama, T., Miyamoto, Y., Nakamura, T., Tamai, Y., Okada, H., Sugiyama, E., Nakamura, T., Itadani, H., and Tanaka, K. 2002. Identification of membrane-type receptor for bile acids (M-BAR). Biochem. Biophys. Res. Commun. 298: 714-719.

### DISCLOSURE OF THE INVENTION

### PROBLEMS THAT THE INVENTION IS TO SOLVE

Recently, the present inventors have identified a novel G-protein-coupled bile acid receptor 1 (Gpbar1) (Non-Patent Reference 17), Gpbar1 is intrinsically expressed in enteroendocrine cell lines such as NCI-H716, STC-1 and GLUTag. It has been known that, in Gpbar1-expressing cells, bile acid does not activate FXR, a nuclear receptor for bile acid, but stimulates cAMP response. Further, since Gpbar1 has been identified, the presence of double signal systems of bile acid, a system via GPCR and a system via nuclear receptor, has been clarified.

However, the accurate role of Gpbar1 in intestines and disorders to be caused by Gpbar1 deficiency are not as yet clarified.

The present invention has been made in consideration of the situation as above, and its object is to clarify the physiological role of Gpbar1 in intestines and to apply the findings to medical care.

More concretely, the invention is to provide a drug for diseases accompanying changes in total bile acid pool or for lipid metabolism disorders, and to provide a screening method for the drug. The invention also provides a test method and a test reagent for diseases accompanying changes in total bile acid pool or for lipid metabolism disorders. Further, the invention provides a genetically-modified non-human mammal in which the expression of a Gpbar1 gene is artificially inhibited.

### MEANS FOR SOLVING THE PROBLEMS

To solve the above-mentioned problems, we, the present inventors have first constructed Gpbar1-deficient mice by destructing the Gpbar1 gene in the mice through homologous recombination, for the purpose of clarifying the *in-vivo* physiological role of Gpbar1. Then, we have measured the total bile acid pool and the fecal bile acid level of the Gpbar1-deficient mice, and have investigated whether or not Gpbar1 participates in the regulation of bile acid homeostasis.

As a result, we have found that, in the homozygous mice, the fecal bile acid level does not change and the total bile acid pool significantly decreases by from 21 to 25 %, as compared with wild-type mice. These suggest that Gpbar1 contributes to the regulation of bile acid homeostasis, indicating that the analysis of Gpbar1-deficient mice is useful for clarifying a novel physiological role of bile acid.

Next, we fed the Gpbar1-deficient mice with high-fat feed, and investigated whether or not Gpbar1 participates in the regulation of lipid metabolism. As a result, we have found that the body weight of the homozygous mice remarkably increases as compared with that of wild-type mice similarly fed with the same high-fat feed. We have found that the body weight increase indicates the increase in fat, and it suggests that the Gpbar1 deficiency causes the abnormality of lipid metabolism.

Since Gpbar1 has relation to the changes in total bile acid pool and to the lipid metabolism abnormality, we have hit on the possibility that drugs for treatment or prevention of diseases accompanying changes in total bile acid pool or lipid metabolism disorders may be specifically identified by screening them on the basis of their bindability to Gpbar1, the expression level of Gpbar1 and the activity of Gpbar1.

Specifically, we, the present inventors have succeeded in developing drugs for diseases that accompany changes in total bile acid pool or for lipid metabolism disorders, and a method of screening these drugs. Further, we have succeeded in developing a test method and a test reagent for diseases accompanying changes in total bile acid pool or for lipid metabolism disorders, and in developing a genetically-modified non-human mammal in which the expression of a Gpbar1 gene is artificially inhibited. On the basis of these, we have completed the present invention.

Specifically, the screening method of the invention for candidate compounds for a drug for treatment or prevention of diseases that accompany a decrease in total bile acid pool or lipid metabolism disorders comprises (a) a step of contacting a test compound with Gpbar1, (b) a step of detecting the binding of the test compound to the Gpbar1, and (c) as step of selecting the test compound that binds to the Gpbar1. According to the method, a compound capable of binding to Gpbar1 and capable of exhibiting the same physiological action as that of bile acid (e.g., Gpbar1 agonist) may be selected. The compound of which the activity is recognized according to the screening method may be a candidate for a remedial or preventive drug for diseases accompanying changes in total bile acid pool or for lipid metabolism disorders.

The screening method may comprise (a) a step of contacting a test compound to a cell that expresses Gpbar1, (b) a step of determining the expression level of the Gpbar1, and (c) a step of selecting the test compound that increased the expression level of the Gpbar1 as compared with a case not contacted with the test compound. According to the method, even a compound not directly reacting with Gpbar1 but capable of reacting with any molecule in a cell to promote the expression of Gpbar1 may be selected.

The screening method may comprise (a) step of providing a cell or cell extract having a DNA of such that a reporter gene functionally binds to the downstream of the promoter region of a Gpbar1-encoding DNA, (b) a step of contacting a test compound with the cell or cell extract, (c) a step of determining the expression level of the reporter gene in the cell or cell extract, and (d) a step of selecting the test compound that increased the expression level of the reporter gene as compared with a case not contacted with the test compound. According to the method, even a compound not directly reacting with Gpbar1 but capable of reacting with the promoter of Gpbar1 to promote the expression of Gpbar1 may be selected.

The screening method may comprise (a) a step of contacting a test compound with a cell that has expressed Gpbar1 on the cell surface, in the presence of a ligand to Gpbar1, (b) a step of determining the activity of Gpbar1 in the cell, and (c) a step of selecting the test compound that increased the activity, as compared with a case not contacted with the test compound. According to the method, a compound having an activity to further promote the activity of Gpbar1 in the presence of a ligand to Gpbar1 may be selected.

The screening method may comprise (a) a step of administering a test compound to a genetically-modified non-human mammal in which the expression of a Gpbar1 gene is artificially inhibited, (b) a step of determining the total bile acid pool in the genetically-modified non-human mammal, and (c) a step of selecting the compound that increased the total bile acid pool in the genetically-modified non-human mammal, as compared with a case not administered with the test compound. According to the method, a compound effective for promoting *in-vivo* Gpbar1 expression or a compound capable of increasing total bile acid pool not via Gpbar1 may be selected, and it may be assessed in point of the presence or absence of its drug potency.

The invention further provides a genetically-modified non-human mammal in which the expression of a Gpbar1 gene is artificially inhibited. The non-human mammal may be used for screening for a compound effective for promoting *in-vivo* Gpbar1 expression or a compound capable of increasing total bile acid pool not via Gpbar1.

The genetically-modified non-human mammal may be constructed by inserting an exogenous gene into one or both of the gene pair of a Gpbar1 gene.

The invention also provides a genetically-modified mammal cell in which the expression of a Gpbar1 gene is artificially inhibited. The genetically-modified mammal cell may be used in screening candidate compounds for drugs for treatment or prevention of diseases that accompany decreases in total bile acid pool or lipid metabolism disorders.

The genetically-modified mammal cell may be a cell derived from a genetically-modified mammal in which an exogenous gene is inserted into one or both of the gene pair of a Gpbar1 gene.

The invention also provides a drug for treatment or prevention of diseases that accompany a decrease in total bile acid pool or lipid metabolism disorders, which comprises, as the active ingredient thereof, a DNA coding for a Gpbar1 protein. When the drug is administered to a patient and when a Gpbar1 protein is produced from the DNA, then diseases that accompany decreases in total bile acid pool or lipid metabolism disorders may be treated or prevented.

Further, the screening method of the invention for candidate compounds for drugs for treatment or prevention of diseases that accompany an increase in total bile acid pool or lipid metabolism disorders comprises (a) a step of contacting a test compound with Gpbar1, (b) a step of detecting the binding of the test compound to the Gpbar1, and (c) a step of selecting the test compound that binds to the Gpbar1. According to the method, a compound capable of binding to Gpbar1 to retard the physiological action of bile acid (e.g., Gpbar1 antagonist) may be selected. The compound of which the activity is recognized through the screening may be a candidate for a remedial or preventive drug for diseases accompanying increases in total bile acid pool or for lipid metabolism disorders.

The screening method may comprise (a) a step of contacting a test compound to a cell that expresses Gpbar1, (b) a step of determining the expression level of the Gpbar1, and (c) a step of selecting the test compound that decreased the expression level of the Gpbar1 as compared with a case not contacted with the test compound. According to the method, even a compound not directly reacting with Gpbar1 but capable of reacting with any molecule in a cell to inhibit the expression of Gpbar1 may be selected.

The screening method may comprise (a) step of providing a cell or cell extract having a DNA of such that a reporter gene functionally binds to the downstream of the promoter region of a Gpbar1-encoding DNA, (b) a step of contacting a test compound with the cell or cell extract, (c) a step of determining the expression level of the reporter gene in the cell or cell extract, and (d) a step of selecting the test compound that decreased the expression level of the reporter gene as compared with a case not contacted with the test compound. According to the method, even a compound not directly reacting with Gpbar1 but capable of reacting with the promoter of Gpbar1 to inhibit the expression of Gpbar1 may be selected.

The screening method may comprise (a) a step of contacting a test compound with a cell that has expressed Gpbar1 on the cell surface, (b) a step of determining the activity of Gpbar1 in the cell, and (c) a step of selecting the test compound that decreased the activity, as compared with a case not contacted with the test compound. According to the method, a compound having an activity to inhibit the activity of Gpbar1 may be selected.

The invention also provides a drug for treatment or prevention of diseases that accompany an increase in total bile acid pool or lipid metabolism disorders, which comprises, as the active ingredient thereof, a compound that retards the expression or the activity of Gpbar1. When the drug is administered to a patient, then diseases accompanying increases in total bile acid pool or lipid metabolism disorders may be treated or prevented.

The invention also provides a drug for treatment or prevention of diseases accompanying changes in total bile acid pool or lipid metabolism disorders, which is selected according to the above-mentioned screening methods. The drug may promote or inhibit the signal transduction at the downstream of Gpbar1 according to a new mechanism unknown up to the present, and may treat or prevent diseases accompanying changes in total bile acid pool or lipid metabolism disorders.

The invention also provides a test method for diseases accompanying changes in total bile acid pool or lipid metabolism disorders, which comprises a step of determining the amount of Gpbar1 gene expression. According to the test method, a small amount of a biological material (e.g., blood) may be tested for diseases accompanying changes in total bile acid pool or lipid metabolism disorders.

The test method preferably comprises a step of detecting the mutation in a Gpbar1 gene region.

In the test, usable is a test reagent for diseases accompanying changes in total bile acid pool or lipid metabolism disorders, which contains an oligonucleotide capable of hybridizing with a Gpbar1 gene region and having a chain length of at least 15 nucleotides.

The test method may contain an antibody that binds to Gpbar1. Even a test reagent that contains an antibody capable of binding to Gpbar1 may be applied to a small amount of a biological material (e.g., blood) for testing it for diseases accompanying changes in total bile acid pool or lipid metabolism disorders.

### EFFECT OF THE INVENTION

In the invention, target disruption to Gpbar1 in mice results in decrease in total bile acid pool, and this suggests that Gpbar1 contributes to the regulation of *in-vivo* bile acid homoeostasis.
Further, when a Gpbar1-deficient mouse is fed with high-fat feed, the body weight of the mouse remarkably increases as compared with a wild-type mouse fed with the same high-fat feed, and this suggests that the Gpbar1 deficiency results in the abnormality of lipid metabolisms.

These lead to the possibility that drugs for treatment or prevention of diseases accompanying changes in total bile acid pool or lipid metabolism disorders may be screened on the basis of their bindability to Gpbar1, the expression level of Gpbar1 and the activity of Gpbar1. Further, on the basis of the expression level of Gpbar1 or the mutation of the Gpbar1 gene therein, samples may be tested for diseases accompanying changes in total bile acid pool or lipid metabolism disorders.

Analysis of the Gpbar1-deficient mouse of the invention is useful for studies of analyzing the physiological role of Gpbar1. The Gpbar1-deficient mouse may be used in presuming the side effect of the drug specifically identified according to the screening method of the invention or that of the Gpbar1 inhibitor such as an anti-Gpbar1 antibody or Gpbar1-antagonist low molecules. Using a cell line established from the tissue of a genetically-modified animal makes it possible to investigate in detail the side effect of the drugs specifically identified according to the screening method in the tissue.

In the genetically-modified animal of the invention, the Gpbar1 gene is inactivated by nature, and therefore the animal may efficiently produce an antibody to a protein that binds to Gpbar1.

Further, when the condition of the genetically-modified animal of the invention is observed and when it is compared with the condition of a disorder of which the cause is not as yet clarified, it is possible to clarify that the cause of the disorder is Gpbar1 dysfunction.

### BRIEF DESCRIPTION OF THE DRAWINGS

[Fig. 1] It is graphs showing mouse Gpbar1 mRNA distribution in tissues.
[Fig. 2] It is a drawing and photographs showing targeted disruption of a mouse Gpbar1 gene.
[Fig. 3] It is graphs showing total bile acid pool and fecal bile acid level in Gpbar1-deficient mice.
[Fig. 4] It is graphs showing body weight change of Gpbar1-deficient mice fed with ordinary feed.
[Fig. 5] It is graphs showing body weight change of Gpbar1-deficient mice fed with high-fat feed.
[Fig. 6] It is graphs showing the fat level and the body weight except fat of Gpbar1-deficient mice fed with high-fat feed.

### BEST MODE FOR CARRYING OUT THE INVENTION

The invention relates to a screening method for candidate compounds for drugs for treatment or prevention of diseases accompanying changes in total bile acid pool or lipid metabolism disorders.

Bile acid is a component of bile, and is synthesized from cholesterol in a liver. This emulsifies fat and assists digestion and absorption in small intestines, and has relation to absorption of various vitamins. This is discharged to intestines via a bile duct, but is almost re-absorbed by the intestinal tract of a terminal ileum and around it, and after having passed through a portal vein, it goes to a liver. In that manner, bile acid undergoes extremely closed enterohepatic circulation.

In the invention, the diseases accompanying changes in total bile acid pool may be any of diseases that accompany decrease in total bile acid pool and diseases that accompany increase in total bile acid pool. The diseases that accompany decrease in total bile acid pool include digestion insufficiency, hormone decrease by cholesterol depression, and injuries of cell membranes of red cells and blood vessels. The diseases that accompany increase in total bile acid pool include arteriosclerosis caused by increase in blood cholesterol.

In the invention, the lipid metabolism disorders may be any disorders caused by the abnormality of lipid metabolism. The diseases based on lipid accumulation caused by the abnormality of lipid metabolisms include, for example, metabolic syndromes such as obesity, diabetes, hyperlipemia, hypertension.

In the first embodiment of the screening method of the invention, plural test compounds are contacted with Gpbar1.

In the invention, the base sequence of a human-derived Gpbar1 cDNA is shown as SEQ ID NO:1; and the amino acid sequence of the protein that the DNA codes for is as SEQ ID NO:2. In addition, the base sequence of a mouse-derived Gpbar1 cDNA is shown as SEQ ID NO:3; and the amino acid sequence of Gpbar1 that the cDNA codes for is as SEQ ID NO:4. Further, the base sequence of a rat-derived Gpbar1 cDNA is shown as SEQ ID NO:5; and the amino acid sequence of Gpbar1 that the cDNA codes for is as SEQ ID NO:6. In this description, Gpbar1 is meant to indicate all of human Gpbar1, mouse Gpbar1 and rat Gpbar1, unless otherwise specifically indicated.

Gpbar1 used in the method of the invention includes a protein that is functionally equivalent to the above-mentioned known Gpbar1 protein. The protein of the type includes, for example, mutants, alleles, variants and homologues of Gpbar1 protein, and fused proteins with partial peptide of Gpbar1 or with any other protein, to which, however, the invention should not be limited.

The mutant of Gpbar1 in the invention includes naturally-derived proteins that comprise an amino acid sequence modified from the amino acid sequence of SEQ ID NO:2, 4 or 6 through substitution, deletion, insertion and/or addition of one or more amino acids therein and functionally equivalent to the protein that comprises the amino acid sequence of SEQ ID NO:2, 4 or 6. In addition, a protein which is coded for by a naturally-derived DNA capable of hybridizing with a DNA that comprises the base sequence of SEQ ID NO:1, 3 or 5 under a stringent condition, and which is functionally equivalent to the protein comprising the amino acid sequence of SEQ ID NO:2, 4 or 6 is also another example of the mutant of Gpbar1.

In the invention, the number of the amino acids to be mutated is not specifically limited, but in general, it may be at most 30 amino acids, preferably at most 15 amino acids, more preferably at most 5 amino acids (e.g., at most 3 amino acids). It is desirable that the amino acid residue to be mutated is mutated to another amino acid of which the property of the amino acid side chains is kept as such. For example, regarding the property of the amino acid side chains, they include hydrophobic amino acids (A, I, L, M, F, P, W, Y, V); hydrophilic amino acids (R, D, N, C, E, Q, G, H, K, S, T); aliphatic side chain-having amino acids (G, A, V, L, I, P); hydroxy-containing side chain-having amino acids (S, T, Y); sulfur atom-containing side chain-having amino acids (C, M); carboxylic acid and amido-containing side chain-having amino acids (D, N, E, Q); base-containing side chain-having amino acids (R, K, H); aromatic group-containing side chain-having amino acids (H, F, Y, W). (The capital letters of the alphabet in the parentheses are one-letter indications of amino acids.) It is known that a polypeptide that has an amino acid sequence modified from its original amino acid sequence through deletion, addition and/or substitution of one or plural amino acid residues therein with any other amino acid still keeps its original biological activity.

In the invention, "functionally equivalent" means that an objective protein has a biological function or a biochemical function equivalent to that of the intended protein. In the invention, the biological function or the biochemical function of the intended protein includes the bindability to bile acid. The biological property includes the specificity to the expression site and the expression level.

A method well known to those skilled in the art for preparing a DNA that codes for "a protein functionally equivalent to" the intended protein is, for example, a method that utilizes a hybridization technique or a polymerase chain reaction (PCR) technique. Specifically, anyone skilled in the art can usually do isolation of a DNA having a high homology to Gpbar1, using the base sequence of Gpbar1 (SEQ ID NO:1, 3 or 5) or a part thereof as a probe or using an oligonucleotide capable of specifically hybridizing with Gpbar1 (SEQ ID NO:1, 3 or 5) as a primer. To that effect, the DNA that codes for a protein having a function equivalent to that of Gpbar1 isolatable through a hybridization technique or a PCR technique is also within the scope of the DNA of the invention.

For such DNA isolation, the hybridization is preferably effected under a stringent condition. The stringent condition for hybridization in the invention indicates a condition of 6 M urea, 0.4 % SDS and 0.5 × SSC, or a hybridization condition of which the stringency is equivalent to that of the former condition. When a condition of higher stringency, for example, a condition of 6 M urea, 0.4 % SDS and 0.1 × SSC is employed, then isolation of a DNA having a higher homology may be expected. The DNA isolated in that manner may have a high homology to the amino acid sequence of the intended protein on the amino acid level. High homology means that the amino acid sequence has the sequence homology in a ratio of at least 50 %, more preferably at least 70 %, even more preferably at least 90 % (for example, at least 95 %, 96 %, 97 %, 98 %, 99 %) of the overall amino acid sequence. The amino acid sequence or base sequence homology may be determined by the use of Karlin & Altschul's algorithm BLAST (Proc. Natl. Acad. Sci. USA 87:2264-2268, 1990; Proc. Natl. Acad. Sci. USA 90: 5873, 1993). A program referred to as BLASTIN or BLASTX has been developed, based on the BLAST algorithm (Altschul SF, et al: J. Mol. Biol. 215: 403, 1990). In case where BLASTN is used for base sequencing, the parameter is, for example, score = 100, word length = 12. In case where BLASTX is used for amino acid sequencing, the parameter is, for example, score = 50, word length = 3. In case where BLAST and gapped BLAST programs are used, the default parameter of each program is used. Concrete processes of these analytic methods are known.

The biological species from which Gpbar1 for use in the method of the invention is derived is not limited to a specific biological species. For example, it includes human, monkey, mouse, rat, guinea pig, porcine, bovine, yeast, insect, etc.

The condition of Gpbar1 to be used in the first embodiment is not specifically defined. For example, it may be in a purified condition, or a condition expressed in a cell, or a condition expressed in a cell extract.

Gpbar1 may be purified in any well-known method. Cells that express Gpbar1 include those expressing endogenous Gpbar1 and those expressing exogenous Gpbar1. The cells expressing endogenous Gpbar1 include cultured cells, which, however, are not limitative. The cultured cells are not specifically defined, and, for example, they may be commercially available. The biological species from which the cells expressing endogenous Gpbar1 are derived is not specifically defined, including human, monkey, mouse, rat, guinea pig, porcine, bovine, yeast, insect, etc. The cells expressing exogenous Gpbar1 may be constructed, for example, by introducing a vector that contains a Gpbar1-encoding DNA into cells. The vector may be introduced into cells in any ordinary method, for example, a calcium phosphate precipitation method, an electric pulse perforation method, a lipofectamine method, a microinjection method, etc. The cells having exogenous Gpbar1 may be constructed, for example, by inserting a Gpbar1-encoding DNA into a chromosome according to a transgenic method that utilizes homologous recombination. The biological species to give the cells for exogenous Gpbar1 introduction thereinto is not limited to mammals, but may be any one for which the technique of intracellular expression of a foreign protein has been established.

The cell extract that expresses Gpbar1 includes, for example, one obtained by adding a vector that contains a Gpbar1-encoding DNA, to a cell extract contained in an *in-vitro* transfer/translation system. The *in-vitro* transfer/translation system is not specifically defined, and may be any commercially-available *in-vitro* transfer/translation kit, etc.

The "test compound" in the method of the invention is not specifically defined, including, for example, single compounds such as natural compounds, organic compounds, inorganic compounds, proteins, peptides; and compound library/gene library expression products, cell extracts, cell culture supernatants, microorganism fermentation products, marine organism extracts, vegetable extracts, procaryotic cell extracts, eucaryotic cell extracts or animal cell extracts. The test sample may be suitably labeled. The labeling includes, for example, radiolabeling, fluorescein-labeling. In addition to the above-mentioned test samples, also usable herein are mixtures of two or more different types of those test samples.

"Contact" in the invention may be attained in any desired manner, depending on the condition of Gpbar1. For example, when Gpbar1 is in a purified condition, then a test sample may be added to the purified product. When it is in such a condition that is it expressed in a cell or in a cell extract, then a test sample may be added to the cell culture or the cell extract. When the test sample is a protein, then, for example, a vector that contains a DNA coding for the protein may be introduced into a Gpbar1-expressing cell, or the vector may be added to a Gpbar1-expressing cell extract. In addition, a two-hybrid assay with, for example, an yeast or animal cell is also employable herein.

In the first embodiment, the binding of the test compound to Gpbar1 is then detected. The detection method is not specifically defined. The binding of the test compound to Gpbar1 may be detected, for example, through labeling given to the test compound bound to Gpbar1 protein (for example, labeling that enables quantitative determination, such as radiolabeling, fluorescein-labeling). In addition, based on the Gpbar1 activity change caused by the binding of the test compound to Gpbar1, the binding of the two may be detected.

In this embodiment, the test compound binding to Gpbar1 is then selected. The selected compound includes a compound that promotes or inhibits the activity of Gpbar1, or a compound that increases or decreases the expression of Gpbar1; and those compounds cause, as a result, the increase or decrease in total bile acid pool.

In the second embodiment of the screening method of the invention, a test compound is first contacted with a cell that expresses Gpbar1.

In the second embodiment, the Gpbar1 expression level is then determined. The Gpbar1 expression level determination may be effected in any method known to those skilled in the art. For example, the mRNA of the gene is extracted according to an ordinary method, and through a northern hybridization method or a RT-PCR method using the mRNA as a template, the gene transfer level may be determined. Further, using a DNA array technique, the gene expression level may also be determined.

A fraction that contains Gpbar1 coded for by the gene is collected according to an ordinary method, and the Gpbar1 expression is detected through electrophoresis such as SDS-PAGE, thereby enabling translation level determination of the gene. Using an antibody to Gpbar1, a western blotting method may be carried out to detect the Gpbar1 expression, thereby enabling translation level determination of the gene.

Not specifically defined, the antibody for use in Gpbar1 detection may be any detectable one. For example, both a monoclonal antibody and a polyclonal antibody may be used herein. The antibody may be prepared in any method known to those skilled in the art. The polyclonal antibody may be obtained, for example, as follows: A small animal such as rabbit is immunized with Gpbar1, or with a recombinant protein expressed in a microorganism such as *E. coli* as a fused protein with GST, or with its partial peptide, and its serum is collected. This is purified, for example, through ammonium sulfate precipitation, protein A, protein G column, DEAE ion-exchange chromatography, or Gpbar1 or synthetic peptide-coupled affinity column, thereby preparing the intended polyclonal antibody. The monoclonal antibody may be obtained, for example, as follows: A small animal such as mouse is immunized with Gpbar1 or its partial peptide, a spleen is taken out of the mouse, this is triturated to separate the cells, then the cells are fused with mouse myeloma cells using a reagent such as polyethylene glycol, and from the fused cells (hybridoma), the clone that produces an antibody to bind to Gpbar1 is selected. Next, the thus-obtained hybridoma is transplanted into the abdomen of a mouse, then ascites is collected from the mouse, and the obtained monoclonal antibody is purified through ammonium sulfate precipitation, protein A, protein G column, DEAE ion-exchange chromatography, or Gpbar1 or synthetic peptide-coupled affinity column, thereby preparing the intended monoclonal antibody.

In the second embodiment, next, the test compound that decreased or increased the Gpbar1 expression level as compared with a case not contacted with the test compound is selected. The selected compound includes a compound that increases or decreases Gpbar1 expression, and the compound causes, as a result, the increase or decrease in total bile acid pool.

In the third embodiment of the screening method of the invention, first provided is a cell or cell extract having a DNA of such that a reporter gene functionally binds to the downstream of the promoter region of a Gpbar1-encoding DNA.

In the third embodiment, "functionally binds" means that a transfer factor binds to the promoter region of a Gpbar1 gene whereby the promoter region of the Gpbar1 gene binds to the reporter gene so as to induce the expression of the reporter gene. Accordingly, even a case where a reporter gene binds to any other gene to form a fused protein with the other gene product may be within the scope of the meaning of the above-mentioned "functional binds" so far as a transfer factor binds to the promoter region of the Gpbar1 gene, thereby inducing the expression of the fused protein.

Not specifically defined, the reporter gene may be any one of which the expression is detectable. For example, it includes a CAT gene, a lacZ gene, a luciferase gene, a β-glucuronidase gene (GUS) and a GFE gene generally used by those skilled in the art. The reporter gene also includes a DNA that codes for Gpbar1 protein.

The cell or cell extract having a DNA of such that a reporter gene functionally binds to the downstream of the promoter region of a Gpbar1-encoding DNA may be prepared according to the method described hereinabove in the section of the first embodiment.

In the third embodiment, next, a test compound is contacted with the cell or cell extract. Next, the expression level of the reporter gene in the cell or cell extract is determined.

The expression level of the reporter gene may be determined in any method known to those skilled in the art, depending on the type of the reporter gene used. For example, in case where the reporter gene is a CAT gene, the reporter gene expression level may be determined by detecting the acetylation of chloramphenicol by the gene product. In case where the reporter gene is a lacZ gene, the coloration of a dye compound by the catalytic action of the gene expression product may be detected; in case where it is a luciferase gene, the fluorescence of a fluorescent compound by the catalytic action of the gene expression product may be detected; in case where it is a β-glucuronidase gene (GUS), the light emission of Glucuron (by ICN) or the coloration of 5-bromo-4-chloro-3-indolyl-β-glucuronide (X-Gluc) by the catalytic action of the gene expression product may be detected; and in case where it is a GFP gene, the fluorescence from a GFP protein may be detected, whereby the reporter gene expression level may be determined in each case.

In case where a Gpbar1 gene is the reporter, the gene expression level may be determined according to the method described hereinabove in the section of the second embodiment.

In the third embodiment, next, the test compound that decreased or increased the expression level of the reporter gene, as compared with a case not contacted with the test compound, is selected. The selected compound includes a compound that increases or decreases the reporter gene expression level, and the compound causes, as a result, the increase or decrease in total bile acid pool.

In the fourth embodiment of the screening method of the invention, first, a test compound is contacted with a cell that has expressed Gpbar1 on the cell surface, in the presence of a ligand to Gpbar1.

"Ligand" as referred to in this description indicates a molecule such as a random peptide or a variable segment sequence capable of being recognized by a specific receptor. The molecule (or polymer complex) recognized by those skilled in the art may be both a receptor and a ligand. In general, a binding partner having a smaller molecular weight is referred to as a ligand, and a binding partner having a larger molecular weight is referred to as a receptor.

In the fourth embodiment, next, the Gpbar1 activity is determined. The Gpbar1 activity includes a binding activity to bile acid, a cAMP producing activity, and a binding activity to [³⁵S]GTPγS. Next, the test compound that decreased or increased the activity, as compared with a case not contacted with the test compound, is selected. The selected compound includes a compound that increases or decreases the activity of Gpbar1, and the compound causes, as a result, the increase or decrease in total bile acid pool.

The invention relates to a genetically-modified non-human mammal in which the expression of a Gpbar1 gene is artificially inhibited.

"The expression of a Gpbar1 gene is artificially inhibited" in the invention generally indicates a condition that the gene expression is inhibited through genetic mutation such as nucleotide insertion, deletion or substitution in one or both of the gene pair of the Gpbar1 gene. A case where a mutant Gpbar1 protein. of which the function as a normal Gpbar1 protein has been reduced or lost, is expressed is also within the scope of the "Gpbar1 gene expression inhibition". The "inhibition" encompasses not only a case where the Gpbar1 gene expression is completely inhibited but also a case where only the expression of one gene of the gene pair of the gene is inhibited. In the invention, it is desirable that the expression of the Gpbar1 gene is specifically inhibited. Not specifically defined, the site in which the gene mutation exists may be any one capable of inhibiting the gene expression. For example, the site includes an exon site, a promoter site, etc.

In the invention, the animal that is targeted for the Gpbar1 gene modification is generally animals except human, and is preferably rodents such as mouse, rat, hamster, rabbit. Of those, more preferred is mouse. The ES cells that are targeted for the Gpbar1 gene modification in the invention are also preferably those derived from rodents, and more preferably those from mouse. So-called "knockout animals" are within the scope of the genetically-modified animals.

In the genetically-modified non-human animal (this may be referred to as "genetically-modified animal") and the genetically-modified ES cells of the invention, the Gpbar1 gene expression may be artificially inhibited, for example, according to a method of deleting all or a part of the Gpbar1 gene, or a method of deleting all or a part of the Gpbar1 gene expression regulation region. For it, preferred is a method of inserting an exogenous gene into one or both of the gene pair of the Gpbar1 gene, thereby inactivating the Gpbar1 gene. Specifically, in an preferred embodiment of the invention, the genetically-modified animal and the genetically-modified ES cell is characterized in that an exogenous gene is inserted into one or both of the gene pair of the Gpbar1 gene.

The genetically-modified animal of the invention may be constructed by anyone skilled in the art according to generally-known genetic engineering technology. For example, a genetically-modified mouse may be constructed as follows: First, a DNA containing an exon part of a Gpbar1 gene is isolated from a mouse, and a suitable marker gene is inserted into the DNA fragment thereby constructing a targeting vector. The targeting vector is introduced into a mouse ES cell line according to an electroporation method or the like, thereby selecting a cell strain having homologous recombination. As the marker gene to be inserted, preferred is an antibiotic-resistant gene such as a neomycin-resistant gene. In case where such an antibiotic-resistant gene is inserted, the homologous recombination-having cell line may be selected only through incubation in a medium that contains the antibiotic. For more efficient screening, a thymidine kinase gene or the like may be bound to the targeting vector. With that, the cell line having non-homologous recombination may be excluded. Further, homologous recombinants may be tested through PCR or southern blotting, whereby a cell line in which one of the gene pair of the Gpbar1 gene is inactivated may be efficiently obtained.

When the cell line having homologous recombination is selected, there may be any other unknown gene disruption owing to gene insertion, than the homologous recombination site, and therefore it is desirable to use plural clones for chimera production. The cell of the obtained ES cell line is injected into a mouse blastoderm whereby a chimera mouse may be obtained. The chimera mouse is interbred to obtain a mouse in which one of the gene pair of the Gpbar1 gene is inactivated. Further, the mouse is interbred to obtain a mouse in which both of the gene pair of the Gpbar1 gene are inactivated. More concretely, according to the method described in Examples given hereinunder, the genetically-modified mouse of the invention may be constructed. The other animals than mouse, of which the ES cell is established, may also be subjected to genetic modification, like the same method as above.

The ES cell line in which both of the gene pair of the Gpbar1 gene are inactivated may be obtained according to the following method. Specifically, a cell of the ES cell line in which one of the gene pair is inactivated is cultivated in a medium containing a high concentration of an antibiotic, whereby an ES cell line may be obtained in which the other one of the gene pair is also inactivated, or that is, both of the gene pair of the Gpbar1 gene are inactivated. Further, the ES cell line of the type may also be obtained as follows: An ES cell line in which one of the gene pair is inactivated is selected, a targeting vector is again introduced into the cell line, and the cell line having homologous recombination is selected. Preferably, the marker gene to be inserted into the targeting vector differs from the above-mentioned marker gene.

The invention also provides a genetically-modified mammal cell obtained from the genetically-modified non-human animal of the invention. The genetically-modified mammal cell is provided not only as a primary cultured cell but also as a cell line established from it. For establishing the genetically-modified animal-derived cell line of the invention, employable is any known method. For example, for rodents, employable is a method of primary cultivation of fetal cells. Further, the genetically-modified mammal cell of the invention may be an ES cell.

The genetically-modified animal, the genetically-modified mammal cell, the cell line established from it, and the ES cell of the invention may be utilized for analysis of detailed functions of Gpbar1 gene. For example, they may be used for presuming the side effect of Gpbar1 inhibitors such as anti-Gpbar1 antibody or Gpbar1 antagonist low molecules. The genetically-modified mouse obtained in the invention grows normally, not dying at least in its fetal stage, and therefore it may be considered the Gpbar1 inhibitor (antagonist) does not have a fatal side effect. The side effect of the Gpbar1 inhibitor may be presumed through detailed investigation of the genetically-modified animal of the invention. Using the genetically-modified mammal cell and the cell line established from it, the side effect of the Gpbar1 inhibitor in each tissue may be investigated in detail. The cells constituting various body tissues (e.g., blood, nerve, liver, pancreas), which are obtained through differential induction from the ES cell of the genetically-modified mammal of the invention, are favorable for screening of candidate compounds for drugs for treatment or prevention of diseases that accompany decreases in total bile acid pool or lipid metabolism disorders.

In the genetically-modified animal of the invention, the Gpbar1 gene is inactivated by nature, and therefore it may efficiently produce an antibody to the protein that binds to Gpbar1. For example, when the genetically-modified mouse of the invention is immunized with Gpbar1 along with a complete Freund's adjuvant, then a monoclonal antibody or a polyclonal antibody to Gpbar1 may be efficiently produced. In this case, the Gpbar1 for immunization may be a mouse-derived one, or may also be a rat or human-derived one.

When the condition of the genetically-modified animal of the invention is observed and when it is compared with the condition of a disease of which the cause is not as yet clarified, then it may be possible that the cause of the disease could be dysfunction of Gpbar1. For example, the phenotype characteristic of the genetically-modified mouse of the invention or the mouse-derived cell is observed and this is compared with various conditions of human diseases. When at least a half of the conditions of the human disease are seen also in the genetically-modified mouse of the invention, then it may be presumed that the cause of the disease could be dysfunction of Gpbar1. The genetically-modified animal of the invention is usable as a model animal for diseases that accompany decreases in total bile acid pool or for lipid metabolism disorders.

Using the genetically-modified non-human mammal, it may be possible to screen candidate compounds for drugs for treatment or prevention of diseases accompanying decreases in total bile acid pool or lipid metabolism disorders.

In the method, first, a test compound is administered to a genetically-modified non-human mammal in which expression of the Gpbar1 gene is artificially inhibited. The administration of the test compound to the genetically-modified animal may be effected orally or parenterally.

Next, the total bile acid pool in the genetically-modified non-human mammal is measured. For measuring the total bile acid pool, the tissue to be analyzed is first homogenized, and while the tissue is perfused, a predetermined amount thereof is extracted with ethanol. Next, the total bile acid content of the extract is determined according to an enzymatic method as in Kitada et al's disclosure (Kitada, H., Miyata, M., Nakamura, T., Tozawa, A., Honma, W., Shimada, M., Nagata, K., Sinal, C. J., Guo, G. L., Gonzalez, F. J., and Yamazoe, Y. 2003; Protective role of hydroxysteroid sulfotransferase in lithocholic acid-induced liver toxicity; J Biol Chem. 278: 17838-17844). This measurement may also be attained according to the method concretely described in Examples.

In this method, next, the test case is compared with a case not administered with the test compound, whereby the compound that increases the total bile acid pool in the genetically-modified non-human mammal is selected. The selected compound includes a compound that increases total bile acid pool, and it is considered that the compound is useful as a drug for treatment or prevention of diseases that accompany decreases in total bile acid pool or lipid metabolism disorders.

The invention also relates to a drug for treatment or prevention of diseases accompanying changes in total bile acid pool or lipid metabolism disorders, which is selected according to the above-mentioned screening method.

The drug for treatment or prevention of diseases accompanying decreases in total bile acid pool or lipid metabolism disorders includes a drug that comprises, as the active ingredient thereof, a DNA which codes for a Gpbar1 protein. The DNA coding for a Gpbar1 protein is as described hereinabove.

The drug for treatment or prevention of diseases accompanying increases in total bile acid pool or lipid metabolism disorders also includes a drug that comprises, as the active ingredient thereof, a compound which lowers the expression or activity of Gpbar1. The compound which lowers the expression or activity of Gpbar1 may be any one capable of lowering, as a result, the expression or the activity of Gpbar1.

The compound that inhibits the expression of Gpbar1 of the invention includes a complementary RNA to the transfer product of a Gpbar1-encoding DNA, or a ribozyme that specifically cleaves the transfer product. The Gpbar1-encoding DNA includes a DNA comprising the base sequence of SEQ ID NO: 1, 3 or 5; a DNA coding for a protein comprising the amino acid sequence of SEQ ID NO: 2, 4 or 6; and a naturally-derived DNA that codes for a protein comprising an amino acid sequence derived from the amino acid sequence of SEQ ID NO: 2, 4 or 6 through substitution, deletion, addition and/or insertion of one or more amino acids therein.

The wording of "inhibiting the expression of Gpbar1" in the invention includes inhibition of gene transfer and inhibition of translation into protein. In addition, it includes not only complete stopping of DNA expression but also DNA expression reduction.

One embodiment of "complementary RNA to the transfer product of DNA that codes for Gpbar1" in the invention is a complementary antisense RNA to the transfer product of DNA that codes for Gpbar1.

The action of the antisense nucleic acid to inhibit the expression of the target gene includes the following plural factors. Specifically, they are transfer initiation inhibition through triple strand formation; transfer inhibition by hybridization with a site in which an open loop structure is locally formed by RNA polymerase; transfer inhibition by hybridization with RNA being produced; splicing inhibition by hybridization at the conjugate point of intron and exon; splicing inhibition by hybridization with a spliceosome-forming site; nucleus-to-cytoplasm transfer inhibition by hybridization with mRNA; splicing inhibition by hybridization with a capping site or a poly(A)-addition site; translation initiation inhibition by hybridization with a translation initiation factor-binding site; translation inhibition by hybridization with a ribosome-binding site near an initiation codon; peptide chain extension inhibition by hybridization with an mRNA translation region or a polysome-binding site; and gene expression inhibition by hybridization with a nucleic acid-protein interaction site. These inhibit the transfer, splicing or translation process to thereby inhibit the expression of the target gene.

The antisense sequence to be used in the invention may inhibit the expression of the target gene according to any of the above-mentioned actions. As one embodiment, an antisense sequence complementary to the non-translation region near the 5'-terminal of the mRNA of gene is planned, then it may be effective for inhibition of gene translation. However, a sequence complementary to the coding region or to the 3'-side non-translation region may also be usable. In that manner, a DNA that contains an antisense sequence to the sequence not only in the translation region of a gene but also in the non-translation region thereof is also within the scope of the antisense DNA for use in the invention. The antisense DNA to be used is linked to the downstream of a suitable promoter, and preferably a sequence that contains a transfer termination signal on the 3'-side is linked thereto. The DNA thus prepared in that manner may be transformed into a desired plant in any known method. The antisense DNA sequence is preferably complementary to the endogenous gene that the plant to be transformed with it has, or a part thereof, but it may not be completely complementary to it so far as it may effectively inhibit the gene expression. The transferred RNA has complementarity to the transfer product of the target gene, preferably to a degree of at least 90 %, most preferably at least 95 %. In order to effectively inhibit the target gene expression by the use of the antisense sequence, the length of the antisense DNA is at least 15 bases or more, preferably at least 100 bases or more, more preferably at least 500 bases or more. In general, the length of the antisense DNA to be used is shorter than 5 kb, preferably shorter than 2.5 kb.

Another embodiment of "complementary RNA to the transfer product of DNA that codes for Gpbar1" is a dsRNA complementary to the transfer product of DNA that codes for Gpbar1. RNAi means a phenomenon that, when a double-strand RNA (hereinafter this is dsRNA) having the same or similar sequence as or to the target gene sequence is introduced into a cell, then expression of both the introduced exogenous gene and the endogenous target gene is inhibited. When a dsRNA of from about 40 to hundreds base pairs is introduced into a cell, then an RNase III-like nuclease having a helicase domain and referred to as "dicer" cleaves the dsRNA into fragments of about 21 to 23 base pairs each from the 3'-terminal, in the presence of ATP, thereby giving siRNA (short interference RNA). A protein specific thereto binds to the siRNA, thereby forming a nuclease complex (RISC: RNA-induced silencing complex). This complex recognizes the same sequence as siRNA, and binds to it, therefore cleaving the mRNA of the target gene at the center part of the siRNA owing to the RNase III-like enzymatic activity thereof. Apart from this route, the antisense chain of the siRNA binds to mRNA, therefore acting as a primer of an RNA-sensitive RNA polymerase (RsRP) to produce dsRNA. Another route may be taken into consideration in which the dsRNA is again to be a substrate of the dicer, thereby producing an additional siRNA to amplify the action thereof.

The RNA of the invention may be expressed by an antisense code DNA that codes for an antisense RNA to a region of the target gene mRNA, and a sense code DNA that codes for a sense RNA for a region of the target gene mRNA. A dsRNA may be produced from the antisense RNA and the sense RNA.

The expression system of the dsRNA of the invention may be held by a vector in different constitutions. In one constitution, an antisense RNA and a sense RNA are expressed by one and the same vector; and in another constitution, an antisense RNA and a sense RNA are individually expressed by different vectors. For example, in the constitution where an antisense RNA and a sense RNA are expressed by one and the same vector, an antisense RNA expression cassette and a sense RNA expression cassette, each comprising a promoter capable of expressing a short RNA such as polIII-type one, linked to the upstream of an antisense code DNA and a sense code DNA, respectively, are separately constructed; and these cassettes are inserted into a vector in the same direction or in opposite directions, thereby constructing the expression system. In addition, an expression system of a different constitution may also be constructed in which an antisense code DNA and a sense code DNA are oppositely disposed to face each other on different chains. This constitution is provided with one double-strand DNA (siRNA code DNA) comprising a pair of an antisense RNA code chain and a sense RNA code chain, and is provided with a promoter linked to each side of the two chains so as to express the antisense RNA and the sense RNA from each chain. In this case, it is desirable that the 3'-terminal of each chain (antisense RNA code chain, sense RNA code chain) is provided with a terminator in order to evade addition of any superfluous sequence to the downstream of the sense RNA and the antisense RNA. The terminator may have a sequence of at least four continuous A (adenine) bases. In the palindrome-style expression system, it is desirable that the two promoters differ from each other.

In the constitution where an antisense RNA and a sense RNA are expressed by different vectors, for example, an antisense RNA expression cassette and a sense RNA expression cassette, each comprising a promoter capable of expressing a short RNA such as polIII-type one, linked to the upstream of an antisense code DNA and a sense code DNA, respectively, are separately constructed; and these cassettes are held by different vectors, thereby constructing the expression system.

In the RNAi of the invention, an siRNA may be used as the dsRNA. "siRNA" means a double-strand RNA that comprises short chains within a range not exhibiting toxicity in cells, and for example, it may be from 15 to 49 base pairs, preferably from 15 to 35 base pairs, more preferably from 21 to 30 base pairs. In addition, the length of the final double-strand RNA moiety after transfer of the expressed siRNA may be, for example, from 15 to 49 base pairs, preferably from 15 to 35 base pairs, more preferably from 21 to 30 base pairs.

The DNA to be used in RNAi may not be completely the same as the target gene, but has the sequence homology of at least 70 %, preferably at least 80 %, more preferably at least 90 %, most preferably at least 95 %.

The double-strand RNA moiety with RNA's pairing to each other in dsRNA is not limited to a completely-pairing one, but may contain a non-pairing moiety owing to mismatching (the pairing bases are not complementary to each other) or bulging (a base corresponding to one chain is missing). In the invention, the double-strand RNA region where RNA's form a pair in dsRNA may contain both the bulging and the mismatching.

"Regulation of Gpbar1 expression" in the invention may be attained by utilizing a DNA that codes for a ribozyme. Ribozyme means an RNA molecule having a catalytic activity. There are known various ribozymes each having a different activity. Through the study of a ribozyme acting as an enzyme that cleaves RNA, it has become possible to plan a ribozyme for site-specific cleavage of RNA. Ribozymes include group I intron-type ones, those having a size of at least 400 nucleotides such as M1RNA included in RNase P, as well as hammerhead-type or hairpin-type ones having an active domain of 40 nucleotides or so.

For example, the self-cleaving domain of a hammerhead-type ribozyme may cleave 3'-side of C15 of G13U14C15, but for its activity, it is said important that U14 forms a base pair with A, and it is shown that the 15-positioned base may be cleaved not only by C but also A or U. When the base-binding site of a ribozyme is so planned as to be complementary to the RNA sequence near the target site, then a restriction endonuclease-type RNA cleavage ribozyme capable of recognizing a sequence of UC, UU or UA in a target RNA may be constructed. For example, the Gpbar1-encoding region of the invention that is to be an inhibitory target contains plural sites to be targets.

A hairpin-type ribozyme is also useful for the purpose of the invention. A hairpin-type ribozyme is found, for example, in the minus chain of the satellite RNA of a tobacco ring spot virus (J. M. Buzayan, Nature 323:349, 1986). It is shown that the ribozyme may also be so planned that it may act for target-specific RNA cleavage.

The ribozyme that is so planned as to be able to cleave a target is linked to a promoter such as 35S promoter of a cauliflower mosaic virus and to a transfer termination sequence in order that it may be transferred in a vegetable cells. In this case, however, when any superfluous sequence is added to the 5'-terminal or the 3'-terminal of the transferred RNA, then the ribozyme may lose its activity. In such a case, another cis-acting trimming ribozyme may be disposed on the 5'-side or the 3'-side of the ribozyme moiety, for the purpose of accurately cutting out only the ribozyme moiety from the transferred ribozyme-containing RNA (K. Taira et al., (1990) Protein Eng. 3:733; A.M. Dzianottand J.J. Bujarski, (1989) Proc. Natl. Acad. Sci. USA. 86:4823; C.A. Grosshans and R.T. Cech, (1991) Nucleic Acids Res. 19:3875; K. Taira et al., (1991) Nucleic Acids Res. 19:5125). These constitution units may be arranged in tandem so as to be able to cleave plural sites in target gene, thereby further increasing the effect (N. Yuyama et al., Biochem. Biophys. Res. Commun. 186:1271, 1992). Using the ribozyme of the type as above, the transfer product of the target gene of the invention may be specifically cleaved to inhibit the expression of the gene.

In case where a compound isolated according to the screening method of the invention, or a DNA that codes for a Gpbar1 protein, or a compound that lowers the expression or the activity of Gpbar1 is used as a drug for humans or other animals, then the compound may be formulated into a pharmaceutical composition according to a known pharmaceutical formulation method and it may be administered to patients, apart from directly administering the compound itself to patients. For example, tablets optionally coated with sugar, or capsules, elixirs, microcapsules may be orally administered; or injections of germ-free solutions or suspensions with water or nay other pharmaceutically-acceptable liquid may be parenterally administered. For example, combined with a pharmaceutically-acceptable carrier or medium, concretely germ-free water, physiological saline water, vegetable oil, emulsifier, suspending agent, surfactant, stabilizer, fragrance, excipient, vehicle, preservative and/or binder, the compound may be mixed in a unit ratio required for usually-admitted pharmaceutical formulation, thereby producing a pharmaceutical composition containing the compound. The amount of the active ingredient in the thus-prepared compositions should be so defined that the suitable amount thereof within an indicated range could be taken by patients.

Additives that may be mixed with tablets and capsules include, for example, binders such as gelatin, corn starch, tragacanth gum, gum arabic; excipients such as crystalline cellulose; expanding agents such as corn starch, gelatin, alginic acid; lubricants such as magnesium stearate; sweeteners such as sucrose, lactose, saccharine; fragrances such as peppermint, akamono oil, cherry. In case where the formulation unit is a capsule, it may contain an oily carrier such as fat and oil, in addition to the above materials. The germ-free composition for injection may be formulated according to ordinary pharmaceutical formulation, using a vehicle such as distilled water for injection.

The aqueous solution for injection includes an isotonic liquid containing, for example, physiological saline water, glucose and any other auxiliary agent, for example, D-sorbitol, D-mannose, D-mannitol, sodium chloride, and it may be combined with a suitable dissolution promoter, for example, alcohol, concretely ethanol, polyalcohol such as propylene glycol, polyethylene glycol, as well as nonionic surfactant such as polysorbate 80(TM), HCO-50.

The oily liquid includes sesame oil, and soybean oil; and it may be combined with a dissolution promoter such as benzyl benzoate, benzyl alcohol. In addition, it may be further combined with a buffer such as phosphate buffer, sodium acetate buffer; an analgesic agent such as procaine chloride; a stabilizer such as benzyl alcohol, phenol; and an antioxidant. The prepared injection may be generally filled in suitable ampoules.

Administration to patients may be effected in any method known to those skilled in the art, for example, through intra-arterial injection, intravenous injection or subcutaneous injection, or intranasal, transbronchial, intramuscular, percutaneous or oral administration. The dose may vary depending on the body weight and the age of the patient and on the administration route, and anyone skilled in the art could suitably determine a suitable dose. When the compound is coded for by a DNA, then the DNA may be inserted into a vector for gene therapy, and may be used according to gene therapy. The dose and the administration method may vary depending on the body weight, the age and the condition of the patient; and anyone skilled in the art could suitably determine it.

The dose of the compound may vary depending on the condition, but in oral administration, the dose to an adult (body weight, 60 kg) may be generally from about 0.1 to 100 mg, preferably from about 1.0 to 50 mg, more preferably from about 1.0 to 20 mg a day.

In parenteral administration, the dose may also vary depending on the subject for administration, the organ for administration, the condition and the administration route. For example, as injection, it may be favorable to apply the compound as intravenous injection at a dose to an adult (body weight, 60 kg) may be generally from about 0.01 to 30 mg, preferably from about 0.1 to 20 mg, more preferably from about 0.1 to 10 mg or so a day. To the other animals, the dose may be determined, as calculated based on the unit body weight of 60 kg or based on the body surface area of the animal.

The invention relates to a test method for diseases that accompany changes in total bile acid pool or lipid metabolism disorders.

The method is a test method for diseases that accompany changes in total bile acid pool or lipid metabolism disorders, and comprises a step of determining the amount of Gpbar1 gene expression. The Gpbar1 gene expression may be determined according to the method described hereinabove.

In case where the Gpbar1 gene expression increases or decreases, then the total bile acid pool may also increase or decrease, and therefore it is possible to determine whether the disease may be one based on the increase or decrease in total bile acid pool.

Further, the method may be a test method for diseases that accompany changes in total bile acid pool or lipid metabolism disorders, and comprises a step of detecting the mutation in a Gpbar1 gene region.

In the invention, the Gpbar1 gene region means a region that has some influence on the Gpbar1 gene and the Gpbar1 gene expression. Not specifically defined, the region that has some influence on the gene expression is, for example, a promoter region.

The mutation in the invention may be any one that participates in diseases accompanying changes in total bile acid pool or lipid metabolism disorders, not specifically defined in point of the kind and the number thereof. Most cases of the mutation are those for changing the expression level of the gene, or those for changing the properties such as stability of mRNA, or those for changing the activity of the protein that is coded for by the gene, which, however, are not limitative. The type of the mutation includes, for example, deletion, substitution or insertion mutation. The mutation includes a mutation that undergoes amino acid substitution in the amino acid sequence of the protein, and a mutation that does not undergo the amino acid substitution but undergoes base substitution in the base sequence.

Preferred embodiments of the test method that comprises a step of detecting the mutation in a Gpbar1 gene region are described below. However, the method of the invention is not limited to those methods.

In a preferred embodiment of the test method, first, a DNA sample is prepared from a subject person. The DNA sample is extracted from the blood, the skin, the oral mucosa or the hair of a subject person, or from the tissue or the cells collected or taken through operation or biopsy. This may be prepared from the chromosomal DNA or RNA.

In this method, next, the DNA containing a Gpbar1 gene region is isolated. The DNA isolation may be attained, for example, through PCR with a chromosomal DNA or RNA-derived cDNA as the template, using a primer capable of hybridizing with the Gpbar1 gene region-containing DNA.

In this method, next, the isolated DNA is sequenced for its base sequence.

In this method, next, the thus-sequenced base sequence of the DNA is compared with a control. In the invention, the control means a DNA that contains a normal (more frequent, or wild) Gpbar1 gene region. In general, the sequence of the DNA that contains a healthy person's Gpbar1 gene region is considered as normal, the above-mentioned "comparing with a control" generally means that the sample DNA is compared with the sequence of the DNA that contains a healthy person's Gpbar1 gene region.

The mutation may be detected in the invention, also according to the following method. First, a DNA sample is prepared from a subject person. Next, the prepared DNA sample is cleaved with a restriction endonuclease. Next, the DNA fragments are separated according to their size. Next, the size of the detected DNA fragment is compared with a control. In another embodiment of the method, first a DNA sample is prepared from a subject person. Next, the DNA that contains a Gpbar1 gene region is amplified. Next, the amplified DNA is cleaved with a restriction endonuclease. Next, the DNA fragments are separated according to their size. Next, the size of the detected DNA fragment is compared with a control.

The method includes, for example, a method that utilizes restriction fragment length polymorphism (RFLP), or a method of PCR-RFLP. Concretely, when a mutation exists in the recognition site of a restriction endonuclease, or when a base insertion or deletion exists in the DNA fragment resulting from restriction endonuclease treatment, then the size of the fragment after restriction endonuclease treatment is compared with a control. The part containing the mutation is amplified through PCR, and then treated with the corresponding restriction endonuclease, and the mutation may be thereby detected as the difference in the band mobility after electrophoresis. Apart from it, a chromosomal DNA may be treated with the corresponding restriction endonuclease, then subjected to electrophoresis, and thereafter processed for southern blotting with the probe DNA of the invention, thereby detecting the presence or absence of the mutation. The restriction endonuclease to be used may be suitably selected in accordance with the corresponding mutation. According to the method, an RNA prepared from a subject person may be converted into its cDNA with a reverse transferase, then this may be directly cleaved with a restriction endonuclease and thereafter may be subjected to southern blotting, apart from the genome DNA. In addition, the DNA that contains a Gpbar1 gene region may be amplified through PCR using the cDNA as a template, and this may be cleaved with a restriction endonuclease and may be checked for the difference in the mobility of the fragment thereof.

In still another method, first, a DNA sample is prepared from a subject person. Next, a DNA that contains a Gpbar1 gene region is amplified. Further, the amplified DNA is dissociated into a single-strand DNA. Next, the dissociated single-strand DNA is separated on a non-modified gel. The mobility of the isolated single-strand DNA on the gel is compared with a control.

The method is, for example, a PCR-single-strand conformation polymorphism (PCR-SSCP) method (Cloning and polymerase chain reaction - single-strand conformation polymorphism analysis of anonymous Alu repeats on chromosome 11. Genomics. 1992 Jan 1; 12(1): 139-146.; Detection of p53 gene mutations in human brain tumors by single-strand conformation polymorphism analysis of polymerase chain reaction products. Oncogene. 1991 Aug 1; 6(8): 1313-1318.; Multiple fluorescence-based PCR-SSCP analysis with postlabeling; PCR Methods Appl. 1995 Apr 1, 4(5): 275-282). The method has advantages in that its operation is relatively easy and the amount of the test sample may be small; and therefore the method is favorable especially for screening a large number of DNA samples. The principle is as follows: When a double-strand DNA fragment is dissociated into single-strands, then each chain forms a peculiar high-order structure depending on the base sequence thereof. The thus-dissociated DNA chains are subjected to electrophoresis in a denaturant-free polyacrylamide gel, then the complementary single-strand DNAs having the same chain length move to a different site depending on the difference in the high-order structure. The high-order structure of the single-strand DNA varies also by one base substitution, therefore showing a different mobility in polyacrylamide gel electrophoresis. Accordingly, the detection of the change in the mobility makes it possible to detect the existence of the mutation in the DNA fragment through spot mutation, deletion or insertion therein.

Concretely, first, a DNA that contains a Gpbar1 gene region is amplified through PCR. The range to be amplified is preferably a length of generally from 200 to 400 bp or so. Anyone skilled in the art may suitably select the reaction condition for PCR. In PCR, a primer labeled with an isotope such as ³²P, or a fluorescent dye or biotin may be used, whereby the amplified DNA product may be labeled. A substrate base labeled with an isotope such as ³²P, or a fluorescent dye or biotin may be added to a PCR reaction liquid to attain PCR, whereby the amplified DNA product may also be labeled. After the PCR reaction, a substrate base labeled with an isotope such as ³²P, or a fluorescent dye or biotin may be added to the amplified DNA fragment for labeling it, using a Klenow enzyme or the like. Thus obtained, the labeled DNA fragment may be modified by heat, and subjected to electrophoresis with polyacrylamide gel not containing a denaturant such as urea. In this step, a suitable amount (from 5 to 10 % or so) of glycerol may be added to polyacrylamide gel, whereby the condition in separating the DNA fragment may be improved. The condition for electrophoresis varies depending on the property of each DNA fragment. In general, it may be attained at room temperature (20 to 25°C); but when desired separation could not be obtained, then a temperature range of from 4 to 30°C may be investigated for the temperature for the best mobility. After the electrophoresis, the mobility of the DNA fragment is detected and analyzed according to autoradiography with an X-ray film, or using a scanner for fluorescence detection. In case where bands differ in their mobility are detected, then the bands are directly cut out from the gel, again amplified through PCR, and directly sequenced to confirm the presence of mutation. Also in case where a labeled DNA is not used, the gels after electrophoresis may be stained with ethydium bromide or according to a silver staining method, and the bands may be thereby detected.

In still another method, first, a DNA sample is prepared from a subject person. Next, the DNA that contains a Gpbar1 gene region is amplified. Further, the amplified DNA is isolated on a gel in which the concentration of the DNA denaturant gradually increases. Next, the mobility of the separated DNA on the gel is compared with a control.

An example of the method is denaturant gradient gel electrophoresis (DGGE). The method of DGGE comprises subjecting a mixture of DNA fragments to electrophoresis in a polyacrylamide gel in which the denaturant has a concentration gradient, whereby the DNA fragments are separated from each other based on the difference in their instability. When mismatched unstable DNA fragments move to a part of a certain denaturant concentration in the gel, then the DNA sequence around the mismatching is partially dissociated into single chains owing to the instability thereof. The mobility of the thus partially-dissociated DNA fragments is extremely low, and is differentiated from the mobility of the complete double-strand DNA with no dissociation; and therefore the two may be separated from each other. Concretely, a DNA that contains a Gpbar1 gene region is amplified through PCR using a primer of the invention, then this is subjected to electrophoresis in a polyacrylamide gel in which the concentration of the denaturant such as urea gradually increases with the movement of the DNA, and then this is compared with a control. A DNA fragment having mutation may be dissociated into single chains at the position having a lower denaturant concentration, and its mobility becomes extremely low; and therefore the presence or absence of mutation in DNA may be detected by detecting the mobility difference.

In still another method, first provided are a DNA that contains a Gpbar1 gene region, as prepared from a subject person, and a substrate with a nucleotide probe to hybridize with the DNA, fixed thereto.

"Substrate" in the invention means a tabular material to which a nucleotide probe may be fixed. In the invention, the nucleotide includes oligonucleotide and polynucleotide. Not specifically defined, the substrate in the invention may be any one to which a nucleotide probe may be fixed, and is preferably those generally used in DNA array technology. In general, a DNA array comprises thousands of nucleotides printed on a substrate at high density. In general, these DNAs are printed on the surface layer of a non-porous substrate. The surface layer of the substrate is generally formed of glass, for which, however, a porous membrane such as a nitrocellulose membrane may also be used.

In the invention, one example of the method of nucleotide fixation (in array) is an oligonucleotide-based array developed by Affymetrix. In the oligonucleotide array, the oligonucleotide may be synthesized *in situ*. For example, already known are *in-situ* producing methods for oligonucleotides by photolithography (by Affymetrix) and inkjet technology for chemical substance fixation (by Rosetta Inpharmatics); and any of these technologies may be utilized in producing the substrate in the invention.

Not specifically defined, the oligonucleotide probe to be fixed to a substrate may be any one capable of detecting the mutation in a Gpbar1 gene region. Specifically, the probe is, for example, a probe that hybridizes with a DNA including a Gpbar1 gene region. So far as it enables specific hybridization, the nucleotide probe may not be completely complementary to the DNA that includes a Gpbar1 gene region. The length of the nucleotide probe to be fixed to a substrate in the invention may be generally from 10 to 100 bases, preferably from 10 to 50 bases, more preferably from 15 to 25 bases when an oligonucleotide is fixed.

In this method, next, the DNA and the substrate are contacted with each other. In this step, the DNA is hybridized with the nucleotide probe. The reaction liquid and the reaction condition for the hybridization may vary depending on various factors such as the length of the nucleotide probe to be fixed to the substrate, and in general, they may be determined according to a method well known to those skilled in the art.

In this method, next, the intensity of the hybridization between the DNA and the nucleotide probe fixed to the substrate is detected. This detection may be attained, for example, by reading the fluorescence signal with a scanner or the like. In a DNA array, the DNA fixed to a slide glass is referred to as a probe, while on the other hand, the labeled DNA in a solution is referred to as a target. Accordingly, the nucleotide fixed to a substrate is referred to as a nucleotide probe in this description. In this method, the thus-detected hybridization intensity is compared with a control.

The method includes, for example, a DNA array method.

Apart from the above-mentioned method, also employable herein is an allele-specific oligonucleotide (ASO) hybridization method for the purpose of detecting only the mutation at a specific site. An oligonucleotide including a base sequence in which mutation may exist is constructed, and this is hybridized with a DNA. IN that case, when mutation exists, then the hybridization efficiency lowers. This may be detected according to a southern blotting method or a method that utilizes the property of a specific fluorescent reagent to lose its fluorescence through its intercalation in the gap of a hybrid.

In the invention, also employable are a TaqMan PCR method, an AcycloPrime Method, and a MALDI-TOF/MS method. For determining the species of base not depending on PCR, usable are an invader method and an RCA method. These methods are described briefly hereinunder. The methods described herein are all applicable to the determination of the base species at the mutation site in the invention.

### [TaqMan PCR Method]

The principle of TaqMan PCR method is as follows: A TaqMan PCR method is an analytic method that utilizes a primer set capable of amplifying an allele-containing region and a TaqMan probe. The TaqMan probe is so planned that it may hybridize with a region that contains an allele capable of being amplified by the primer set.

When a TaqMan probe is hybridized with a target base sequence under a condition near to Tm of the probe, the hybridization efficiency of the TaqMan probe remarkably lowers owing to one base difference. In PCR in the presence of a TaqMan probe, the extension reaction from the primer soon reaches the hybridized TaqMan probe. In this stage, the TaqMan probe is decomposed from its 5'-terminal by the 5',3'-exonuclease activity of a DNA polymerase. When the TaqMan probe is labeled with a reporter dye and a quencher, then the TaqMan probe decomposition may be traced as the change in the fluorescence signal. In other words, the TaqMan probe decomposition, if any, results in the formation of a fluorescence signal owing to the release of the reporter dye to cause the separation thereof from the quencher. When the TaqMan probe hybridization lowers owing to one base difference, then the TaqMan probe decomposition does not go on and the fluorescence signal is not formed.

When a TaqMan probe corresponding to mutation is so designed that it may produce different signals through each probe decomposition, then the base species may be simultaneously identified. As a reporter dye, for example, 6-carboxy-fluorescein (FAM) is used for the TaqMan probe of allele A of an allele, and VIC is used for the probe of allele B. Under the condition under which the probe is not decomposed, the fluorescence signal formation by the reporter dye is inhibited by a quencher. When each probe has hybridized with the corresponding allele, then a fluorescence signal corresponding to the hybridization is observed. Specifically, in case where any signal of FAM or VIC is stronger than the other, then homo-allele A or allele B is identified. On the other hand, when it has a hetero-allele, then both signals are detected nearly on the same level. Utilizing the TaqMan PCR method enables genome analysis to simultaneously attain both PCR and base species determination, not requiring a time-consuming step of separation on a gel. Accordingly, the TaqMan PCR method is useful for many subject persons for determining their base species.

### [AcycloPrime Method]

As a method of base species determination through PCR, an AcycloPrime method is also practicable. In an AcycloPrime method, used are a pair of primers for genome amplification and one primer for SNPs detection. First, PCR is amplified in a region that contains a mutation site of a genome. This step is the same as ordinary genome PCR. Next, the obtained PCR product is annealed with the primer for SNPs detection, for its chain extension. The primer for SNPs detection is so designed that it may anneal in the region adjacent to the mutation site to be detected.

In this stage, as the nucleotide substrate for chain extension, used is a nucleotide derivative (terminator) labeled with a fluorescent polarizing dye and blocked at the 3'-OH thereof. As a result, only one base complementary to the base at the position corresponding to the mutation site is taken in to terminate the chain extension. The taking of the nucleotide derivative into the primer may be detected by the increase in the fluorescence polarization (FP) owing to the increase in the molecular weight. When two different types of fluorescent polarizing dyes each having a different wavelength are used for the labeling, then the specific SNPs may be identified as any one of the two bases. The level of the fluorescence polarization may be quantified, and therefore one analysis according to the method makes it possible to determine whether the allele is homo or hetero-type one.

### [MALDI-TOF/MS Method]

The base species may also be identified through analysis of PCR product in MALDI-TOF/MS. MALDI-TOF/MS gives a molecular weight extremely accurately, and it is utilized in various fields as a method of analysis of protein for clarifying the amino acid sequence thereof or DNA analysis for clarifying any minor difference in the base sequence thereof. For base species determination according to MALDI-TOF/MS, the region that includes an allele to be analyzed is first amplified through PCR. Next, the amplified product is isolated and its molecular weight is determined through MALDI-TOF/MS. Since the base sequence of the allele is known, the base sequence of the amplified product may be indiscriminately determined based on the molecular weight thereof.

Base species determination through MALDI-TOF/MS requires a step of separating a PCR product. However, accurate base species determination may be expected through it, not using a labeled primer or a labeled probe. In addition, it may be applicable to simultaneous detection of mutation at plural sites.

### [SNSs-specific Labeling Method with IIs-type restriction endonuclease]

A method that enables base species determination at higher speed through PCR is also reported. For example, a IIs-type restriction endonuclease is used for base species determination in a mutation site. In this method, a primer having a IIs-type restriction endonuclease-recognizing sequence is used in PCR. An ordinary restriction endonuclease (type II) used in genetic recombination recognizes a specific base sequence and cleaves a specific site in the base sequence. As opposed to it, a IIs-type restriction endonuclease recognizes a specific base sequence and cleaves a site spaced from the recognized base sequence. The number of bases between the recognized sequence and the cleaved site depends on the enzyme used. Accordingly, when a primer that contains a recognition sequence of a IIs-type restriction endonuclease is so designed that it may anneal with the amplified product at the site thereof spaced by the number of those bases, then the amplified product may be cleaved just at the mutation site by the IIs-type restriction endonuclease.

At the terminal of the amplified product cleaved with a IIs-type restriction endonuclease, formed is a cohesive end that contains a base of SNPs. At this, an adaptor comprising a base sequence corresponding to the cohesive end of the amplified product is ligated. The adaptor comprises different base sequences that contain bases corresponding to the mutation, and they may be labeled with different fluorescent dyes. Finally, the amplified product is labeled with a fluorescent dye corresponding to the base at the mutation site.

When a capture primer is combined with the above-mentioned, IIs-type restriction endonuclease-recognizing sequence-containing primer in PCR, then the amplified product may be fluorescein-labeled and at the same time it may be converted into a solid phase with the capture primer. For example, when a biotin-labeled primer is used as a capture primer, then the amplified product may be captured by avidin-bound beads. The fluorescent dye of the thus-captured, amplified product may be traced to thereby determine the base species.

### [Base Species Determination at a mutation site with magnetofluorescent beads]

Also known is a technique of parallel analysis of plural alleles in a single reaction system. Parallel analysis of plural alleles is referred to as multiplexing. In general, a typing method with a fluorescent signal requires fluorescent components each having a different fluorescence wavelength for multiplexing. However, there are not so many fluorescent components usable in actual analysis. As opposed to it, when plural types of fluorescent components are mixed in a resin, then even limited types of fluorescent components may obtain various fluorescent signals that may be mutually differentiated from each other. Further, when a component capable of being adsorbed by a magnetic power is added to a resin, then it may produce fluorescence-emitting and magnetically-separable beads. A technique of multiplexing mutation typing with such magnetic fluorescent beads has been found out (Bioscience and Bioindustry, Vol. 60, No. 12, 821-824).

In multiplexing mutation typing with magnetic fluorescent beads, a probe that has a base complementary to the mutation site of each allele at its terminal is fixed to magnetic fluorescent beads. The two are so combined that the magnetic fluorescent beads having a fluorescent signal intrinsic to each allele may correspond to the probe. On the other hand, when the probe thus fixed to the magnetic fluorescent beads has hybridized with the complementary sequence, then it forms a fluorescein-labeled oligo-DNA having a base sequence complementary to the adjacent region on the allele.

The allele-containing region is amplified through asymmetric PCR, then the above-mentioned magnetic fluorescent beads-fixed probe is hybridized with the fluorescein-labeled oligo-DNA, whereby the two are ligated. In case where the terminal of the magnetic fluorescent beads-fixed probe is a base sequence complementary to the base of the mutation site, then the two are efficiently ligated. On the contrary, when the terminal base varies owing to mutation, then the ligation efficiency of the two lowers. As a result, only in a case where the sample is a base species complementary to the magnetic fluorescent beads, the fluorescein-labeled oligo-DNA may bind to the respective magnetic fluorescent beads.

The magnetic fluorescent beads are collected by a magnetic power, and the presence of the fluorescein-labeled oligo-DNA on the respective magnetic fluorescent beads is detected, whereby the base species is determined. Every magnetic fluorescent bead may be analyzed for its fluorescence signal, using a flow site meter, and therefore even though various types of magnetic fluorescent beads are mixed, the signal separation is easy. Accordingly, "multiplexing" for parallel analysis of different types of mutation sites in one reaction container is thus attained.

### [Invader Method]

A method for genotyping, not depending on PCR, is also practicable. For example, an invader method realizes base species determination only by three oligonucleotides of allele probe, invader probe and FRET probe, and a special nuclease referred to as cleavage. Of those probes, the FRET probe alone requires labeling.

The allele probe is so designed that it may hybridize with a region adjacent to the allele to be detected. On the 5'-side of the allele probe, a flap that comprises a base sequence irrelevant to hybridization is linked to it. The allele probe is so constituted that it hybridizes at the 3'-side of the mutation site, and it links to the flap on the mutation site.

On the other hand, the invader probe comprises a base sequence that hybridize on the 5'-side of the mutation site. The base sequence of the invader probe is so designed that the 3'-terminal thereof may correspond to the mutation site through hybridization. The base at the position corresponding to the mutation site in the invader probe may be any one. In other words, the base sequences of the two are so designed that the invader probe and the allele probe may hybridize neighboring to each other via the mutation site therebetween.

In case where the mutation site is a base that is complementary to the base sequence of the allele probe, and when both the invader probe and the allele probe hybridize with an allele, then it forms a structure where the invader probe has invaded the base corresponding to the mutation site of the allele probe. The cleavage cleaves the chain on the invaded side of the oligonucleotides having the thus-formed invaded structure. The cleavage occurs on the invaded structure, and as a result, the flap of the allele probe is cleaved away. On the other hand, if the base of the mutation site is not complementary to the base of the allele probe, then there is not competition between the invader probe and the allele probe in the mutation site, and therefore the invaded structure is not formed. Accordingly, flap cleavage by the cleavage does not occur.

The FRET probe is a probe for detecting the thus-cleaved flap. The FRET probe constitutes a hairpin loop that has a self-complementary sequence on the 5'-terminal thereof, and has a single-strand part disposed on the 3'-terminal side thereof. The single-strand part disposed on the 3'-terminal side of the FRET probe comprises a base sequence complementary to the flap, and the flap may hybridize at it. The base sequences of the two are so designed that, when the flap has hybridized with the FRET probe, then it may form a structure in which the 3'-terminal of the flap has invaded the 5'-terminal part of the self-complementary sequence of the FRET probe. The cleavage cleaves it, having recognized the invaded structure. When the area sandwiched between the parts to be cleaved with the cleavage of the FRET probe is labeled with the same reporter dye and quencher as in TaqMan PCR, then the cleavage of the FRET probe may be detected as the change of the fluorescence signal.

Theoretically, the flap may hybridize with the FRET probe though it is not cleaved. In fact, however, the cleaved flap and the flap existing in the allele probe as such therein have a significant difference in the binding efficiency to FRET. Accordingly, using the FRET probe, it is possible to specifically detect the cleaved flap.

For determining the base species based on the invader method, two types of allele probes shall be prepared, including base sequences individually complementary to allele A and allele B. In this, the base sequences of the flaps of the two are different from each other. Also two types of FRET probes for flap detection are prepared, and the respective reporter dyes that are distinguishable from each other are prepared. With that, the base sequence may be determined according to the same idea as in the TaqMan PCR method.

The advantage of the invader method is that only the FRET probe for use therein requires labeling of its oligonucleotide. One and the same oligonucleotide may be used for the FRET probe, irrelevant to the base sequence to be detected. Accordingly, mass-production is applicable to it. On the other hand, labeling is unnecessary for the allele probe and the invader probe, and after all, the reagents for genotyping may be produced at low costs.

### [RCA Method]

An RCA method is mentioned for base species determination not depending on PCR. A DNA amplification method based on the reaction of a DNA polymerase having a chain-substituting action to produce a long complementary chain, using a cyclic single-strand DNA as a template, is a rolling circle amplification (RCA) method (Lizardri PM et al., Nature Genetics 19, 225, 1998). In the RCA method, a primer that anneals with a cyclic DNA to initiate complementary chain synthesis and a second primer that anneals with the long complementary chain formed by the first primer are used for constituting the amplification reaction.

In the RCA method, used is a DNA polymerase having a chain-substituting action. Accordingly, the part that has changed to a double-strand through the complementary chain synthesis is substituted through the complementary chain synthesis reaction initiated from another primer having annealed at a part nearer to the 5'-side. For example, the complementary chain synthesis reaction with a cyclic DNA as a template does not terminate in one circle. The complementary chain synthesis further continues with substituting the previously-synthesized complementary chain, thereby producing a long single-strand DNA. On the other hand, with the long single-strand DNA formed from the cyclic DNA as a template, the second primer anneals to initiate complementary chain synthesis. The single-strand DNA formed in the RCA method is from the cyclic DNA as a template, and therefore its base sequence is a repetition of the same base sequence. Accordingly, continuous production of a long single-strand brings about continuous annealing with the second primer. As a result, not via a denaturation step, the single-strand part with which the primer may anneal is continuously produced. Accordingly, DNA amplification is attained.

When a cyclic single-strand DNA necessary for RCA is formed depending on the base species of the mutation site, then the RCA method may be used for base sequence determination. For this, a linear single-strand padlock probe is used. The padlock probe has base sequences complementary to both sides of the mutation site to be detected, at its 5'-terminal and 3'-terminal. These base sequences are linked to each other via a part comprising a special base sequence referred to as a backbone. When the mutation site is a base sequence complementary to the terminal of the padlock probe, then the terminal of the padlock probe hybridized with an allele may be ligated with a DNA ligase. As a result, the linear padlock probe is cyclized and the reaction for the RCA method is thereby triggered. The efficiency of the DNA ligase reaction is extremely low when the terminal part to be ligated is not completely complementary.
Accordingly, the presence or absence of ligation may be confirmed through RCA, thereby enabling the base sequence determination in the mutation site.

In the RCA method, DNA may be amplified but it does not form a signal as such. When only the presence or absence of amplification is the index of the method, then in general, every allele individually requires its own reaction for base species determination. A method improved in this point for base species determination is known. For example, using a molecular beacon, a base species may be determined in one tube according to the RCA method. The molecular beacon is a signal-forming probe that uses a fluorescent dye and a quencher like in the TaqMan method. The 5'-terminal and the 3'-terminal of the molecular beacon are composed of complementary base sequences, and each forms a hairpin structure by itself. When the parts near to both ends are labeled with a fluorescent dye and a quencher, then the hairpin structure condition does not emit a detectable fluorescent signal. When a part of the molecular beacon is modified to have a base sequence complementary to the amplified product in RCA, then the molecular beacon may hybridize with the amplified product in RCA. The hybridization decomposes the hairpin structure, and a fluorescent signal may be thereby formed.

The advantage of the molecular beacon is that, by utilizing the base sequence of the backbone part of the padlock probe, the molecular beacons may have a common base sequence irrelevant to the subject to be detected. When the backbone sequence is varied depending on every allele and when two types of molecular beacons differing in the fluorescent wavelength for them are combined, then base species determination may be possible in one tube. Since the production costs for fluorescein-labeled probes are high, it is an economical advantage that common probes may be utilized irrespective of the subject to be detected.

These methods have been developed for rapid genotyping of a large quantity of samples. Except MALDI-TOF/MS, in general, all these methods require a probe labeled in any manner. As opposed to these, base species determination not relying upon a labeled probe has been carried out from the past. Such methods include, for example, a method utilizing restriction fragment length polymorphism (RFLP), and a PCR-RFLP method.

In case where Gpbar1 gene mutation is confirmed according to these methods, it is considered that total bile acid pool simultaneously decreases, and it may be judged that the detection indicates a disorder based on the decrease in total bile acid pool. Depending on the mutation position, the total bile acid pool may increase, and in such a case, it may be judged that the detection indicates a disorder based on the increase in total bile acid pool.

The invention relates to a test reagent for diseases that accompany changes in total bile acid pool or lipid metabolism disorders.

The test reagent for diseases accompanying changes in total bile acid pool or lipid metabolism disorders may contain an oligonucleotide capable of hybridizing with a Gpbar1 gene region and having a length of at least 15 nucleotide chains.

The oligonucleotide of the invention includes a polynucleotide. The oligonucleotide of the invention may be used for a probe and a primer for detection and amplification of the DNA that codes for the protein of the invention, for a probe and a primer for detection of the expression of the DNA, and for a nucleotide or nucleotide derivative (for example, antisense oligonucleotide or ribozyme or DNA that codes for these) for regulation of the expression of the protein of the invention. In addition, the oligonucleotide of the invention may be used as a form of a substrate of a DNA array.

In case where the oligonucleotide is used as a primer, its length may be generally from 15 bp to 100 bp, preferably from 15 bp to 30 bp. Not specifically defined, the primer may be any one capable of amplifying at least a part of the DNA or its complementary chain of the invention. In case where it is used as a primer, the 3'-side region thereof may be complementary region and the 5-side thereof may have a restriction endonuclease-recognizing sequence or a tag added thereto.

In case where the above-mentioned oligonucleotide is used as a probe, the probe may be any one, not specifically defined, capable of specifically hybridizing with at least a part of the DNA or its complementary chain of the invention. The probe may be a synthetic oligonucleotide, generally having a chain length of at least 15 bp.

In case where the oligonucleotide of the invention is used as a probe, it is preferably labeled in a suitable manner. Examples of the method of labeling it are a method of using a T4 polynucleotide kinase to phosphorylate the 5'-end of the oligonucleotide with ³²P for labeling it; and a method of using a DNA polymerase such as Klenow enzyme and using a random hexamer oligonucleotide or the like as a primer, thereby taking a substrate base labeled with an isotope such as ³²P, or a fluorescent dye or biotin, into the probe (random prime method).

The oligonucleotide of the invention may be produced, for example, using a commercially-available oligonucleotide synthesizer. The probe may be produced also as a double-strand DNA fragment obtained through restriction endonuclease treatment.

The test reagent for diseases that accompany changes in total bile acid pool or lipid metabolism disorders may contain an antibody binding to Gpbar1.

Not specifically defined, the antibody of the invention may be any one capable of recognizing Gpbar1, but is preferably an antibody specifically recognizing Gpbar1.

Not specifically defined, the antibody to be used for detecting the protein may be any one that enables the detection, and, for example, both of a monoclonal antibody and a polyclonal antibody may be used. For the antibody for recognizing the protein of the invention, usable is any known antibody. The antibody may be produced in any method known to those skilled in the art, using the protein as an antigen.

Concretely, for example, it may be produced as follows:

A small animal such as rabbit is immunized with the protein or a recombinant protein expressed in microorganisms such as E. coli as a fused protein with GST, or its partial peptide, and its serum is collected. This is purified, for example, through ammonium sulfate precipitation, protein A, protein G column, DEAE ion-exchange chromatography, or the protein or synthetic peptide-coupled affinity column, thereby preparing the intended antibody. The monoclonal antibody may be obtained, for example, as follows: A small animal such as mouse is immunized with the protein or its partial peptide, a spleen is taken out of the mouse, this is triturated to separate the cells, then the cells are fused with mouse myeloma cells using a reagent such as polyethylene glycol, and from the fused cells (hybridoma), the clone that produces an antibody to bind to the protein is selected. Next, the thus-obtained hybridoma is transplanted into the abdomen of a mouse, then ascites is collected from the mouse, and the obtained monoclonal antibody is purified through ammonium sulfate precipitation, protein A, protein G column, DEAE ion-exchange chromatography, or the protein or synthetic peptide-coupled affinity column, thereby preparing the intended monoclonal antibody.

The polyclonal antibody may be obtained, for example, as follows: The protein or its fragment is used as a sensitizing antigen. Cells are immunized with it in an ordinary immunization method, and the resulting immunized cell is fused with a known parent cell in an ordinary cell fusion method. The fused cells are screened according to an ordinary screening method to obtain a monoclonal antibody-producing cell (hybridoma). The antigen may be prepared according to a known method, for example, according to a method of using a baculovirus (WO98/46777). The hybridoma may be constructed, for example, according to a Milstein et al's method (Kohler, G. and Milstein, C., Methods Enzymol. (1981) 73: 3-46). In case where the immunogenicity of the antigen is low, the antigen may be bound to a macromolecule having immunogenicity, such as albumin, and may be used for immunization. Next, from the mRNA of the hybridoma, produced is the cDNA of the variable region (V-region) of the antibody, using a reverse transcriptase; and the sequence of the resulting cDNA may be analyzed according to a known method.

Not specifically defined, the antibody that recognizes the protein may be any one that binds to the protein, and any of a mouse antibody, a rat antibody, a rabbit antibody, a sheep antibody and a human antibody may be suitably used for it. In addition, a genetic recombinant antibody artificially modified for the purpose of lowering the hetero-antigenicity to humans, for example, a chimeric antibody, a humanized antibody may also be used. These modified antibodies may be produced according to a known method. The chimeric antibody is, for example, an antibody that comprises a variable region of the heavy chain and the light chain of an antibody of a mammal except human, for example, a mouse antibody, and a constant region of the heavy chain and the light chain of a human antibody; and this may be obtained by linking the DNA that codes for the variable region of a mouse antibody to the DNA that codes for the constant region of a human antibody, followed by inserting it into an expression vector and introducing it into a host to produce the intended chimeric antibody.

The humanized antibody may be referred to also as a reshaped human antibody, and this may be constructed by transplanting the complementarity-determining region (CDR) of an antibody of a mammal except human, for example, a mouse antibody, in the complementarity-determining region of a human antibody, and a general genetic recombination method for it is known. Concretely, a DNA sequence that is so designed as to link the CDR of a mouse antibody to the framework region (FR) of a human antibody is synthesized from a few oligonucleotides formed to have an overlapping part at their ends, through PCR. The obtained DNA is linked to a DNA that codes for the constant region of a human antibody, then inserted into an expression vector, and this is introduced into a host, which produces the intended humanized antibody (see EP-A239400, WO96/02576). FR of the human antibody to be linked via CDR is so selected that the complementarity-determining region may form a good antigen-binding site. If desired, the amino acid in the framework region of the variable region of the antibody may be substituted so that the complementarity-determining region of the reshaped human antibody may form a suitable antigen-binding site (Sato, K, et al., Cancer Res. (1993) 53, 851-856).

A method of obtaining a human antibody is also known. For example, human lymphocytes are *in-vitro* sensitized with the desired antigen or with cells expressing the desired antigen, and the sensitized lymphocytes are fused with human myeloma cells, for example, U266, thereby obtaining a desired human antibody that has a binding activity to the antigen (see JP-B 1-59878). In addition, a transgenic animal having all repertories of human antibody genes may be immunized with a desired antigen, thereby obtaining the desired human antibody (see WO93/12227, WO92/03918, WO94/02602, WO94/25585, WO96/34096, WO96/33735). Further, known is a technique of obtaining a human antibody through panning, using a human antibody library. For example, the variable region of a human antibody may be expressed as a single-strand antibody (scFv) on the surface of a phage according to a phage display method, and the phage binding to the antigen may be selected. The gene of the selected phage is analyzed, whereby the DNA sequence that codes for the variable region of the human antibody binding to the antigen may be determined. After the DNA sequence of scFv that binds to the antigen is clarified, a suitable expression vector having the sequence may be constructed and a human antibody may be thereby obtained.

The antibody to be used in the invention may be a conjugate antibody binding to various molecules such as polyethylene glycol (PEG), radioactive substances, toxins. The conjugate antibody may be constructed by chemically modifying the obtained antibody. The modification method for the antibody has already been established in this technical field. "Antibody" in the invention includes the conjugate antibody.

The above-mentioned test reagent may optionally contain, in addition to the oligonucleotide or the antibody that are active ingredients, for example, germ-free water, physiological saline water, vegetable oil, surfactant, lipid, dissolution promoter, buffer, protein stabilizer (e.g., BSA, gelatin), preservative.

### EXAMPLES

The invention is described more concretely with reference to the following Examples, to which, however, the invention should not be limited.

All values in the following Examples are expressed as mean value ± standard error (SE). These values are analyzed by ANOVA (StatView Windows^{™}, ver. 5.0) associated with a post-hook Bonferroni test.

### [Example 1] Analysis of Tissue Distribution of Mouse Gpbar1 mRNA:

For clarifying the tissue distribution of mouse Gpbar1 mRNA, various mouse tissues were analyzed through quantitative RT-PCR.

Various tissues (brain, lung, heart, liver, spleen, kidney, stomach, jejunum, ileum, colon, skeletal muscle, brown fat tissue and white fat tissue) were taken out from 6 to 10-week age C57BL/6N mice. From these, a total RNA was extracted with ISOGEN (by Nippon Gene, Tokyo). The total RNA derived from each tissue was quantified with a spectrophotometer, and then a predetermined amount of it was reacted with a reverse transcriptase, and the obtained cDNA was quantitatively analyzed through RT-PCR. The quantitative RT-PCR analysis was carried out according to a TaqMan PCR method, using a PRISM 7900HT sequence detector (by Applied Biosystems, USA). For the primer and the probe for detecting mouse Gpbar1 expression, used was an assay on demand set (Assay ID: Mm00558112_s1) bought from Applied Biosystems.

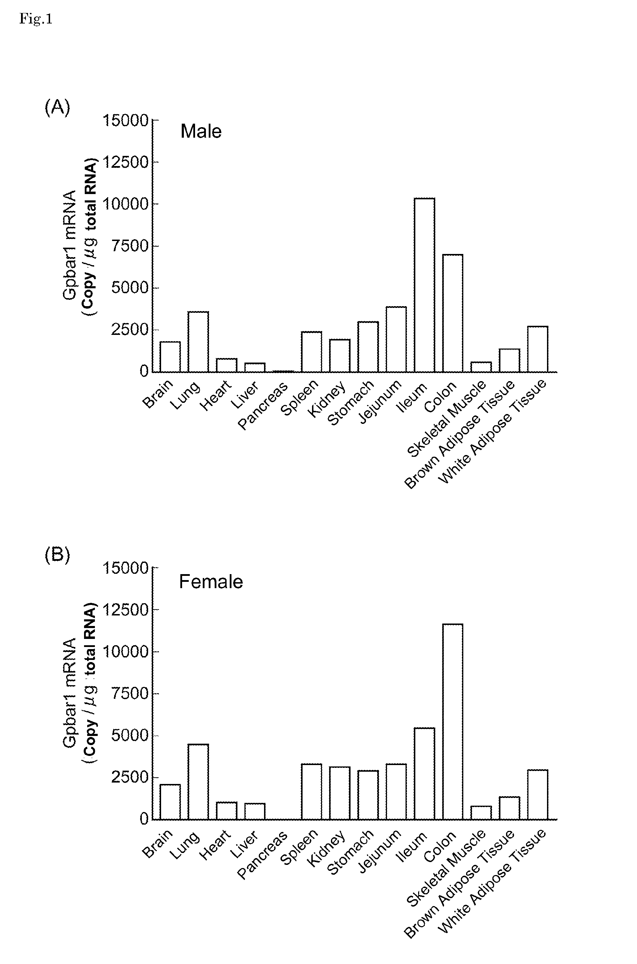

As a result of quantitative RT-PCR, the mouse Gpbar1 mRNA was strongly detected in the ileum and colon of the male mouse and in the colon of the female mouse; and was detected in a moderate degree also in the lung, spleen, kidney, stomach, jejunum and white fat tissue of the mice, irrespective of the sex thereof (Figs. 1A, 1B). In addition, also in the ileum of the female mouse, it was detected in a moderate degree. From this, it has been clarified that Gpbar1 mRNA is strongly expressed in the small intestine and the large intestine, and it is suggested that Gpbar1 plays an important role in the intestines along with bile acid. In addition, since it is known that human GPBAR1 mRNA is also expressed in the small intestine and the large intestine, it is believed that Gpbar1 may play a common role both in humans and mice (Maruyama, T., Miyamoto, Y., Nakamura, T., Tamai, Y., Okada, H., Sugiyama, E., Nakamura, T., Itadani, H., and Tanaka, K.; 2002, Identification of membrane-type receptor for bile acids (M-BAR), Biochem. Biophys. Res. Commun. 298: 714-719). However, human GPBAR1 mRNA is detected in a liver; but the expression level of mouse Gpbar1 mRNA was smaller in a liver than in the other tissues.

### [Example 2] Construction of Gpbar1-Deficient Mouse:

A Gpbar1-deficient mouse was constructed for investigating the *in-vivo* physiological role of Gpbar1.

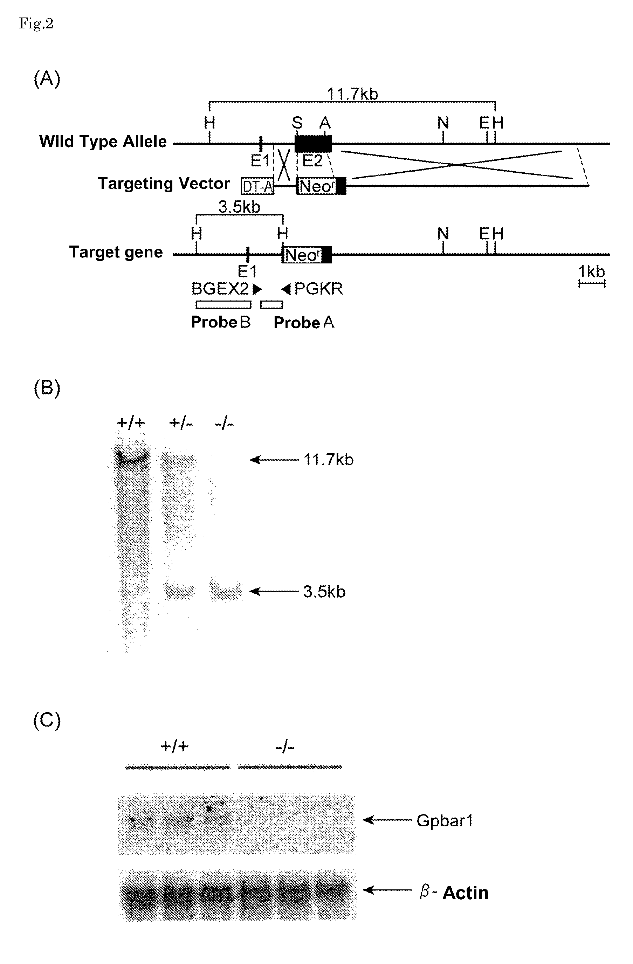

A mouse in which the Gpbar1 gene was target-disrupted was constructed according to the method described below and according to the targeting strategy described in Fig. 2A. In Fig. 2A, the black square part indicates an exon (E1 and E2); and the alphabetical symbols indicate the corresponding restriction endonuclease sites (H is HindIII; S is SphI; A is ApaI; N is NsiI; and E is EcoRI).

First, using a 1.2 kb total cDNA of mouse Gpbar1/M-Bar (GenBank Accession No. AB086170), 129/Sv mouse genome λ phage library (Stratagene) was screened to obtain a mouse genome Gpbar1 clone containing exons 1 and 2. Almost all the exon-2 region containing the ATG codon of Gpbar1 gene was substituted with PGK-neo cassette (Fig. 2A). A targeting vector was made linear at the single SalI site, and introduced into a mouse embryo stem (ES) cell, RW4, according to an electroporation method.

Next, for identifying a homologue recombinant (HR), ES cells transfected with neomycin resistance were screened through PCR to select 672 colonies. Using primers BGEX2 (5'-CAGAGGAGCAGAGGGCAGAATC-3', SEQ ID NO: 7) and PGKR (5'-CTAAAGCGCATGCTCCAGACT-3', SEQ ID NO: 8), these clones were further screened through PCR of 40 cycles, one cycle comprising 94°C for 30 seconds, 68°C for 1.5 minutes, and 72°C for 2 minutes, whereby 7 positive clones were collected. Further, after digested with HindIII, these positive clones were subjected to genome southern blotting analysis using probes A and B, and were thus analyzed.

The homologous recombinant clone was injected into a C57BL/6 blastocyst, and implanted into a pseudopregnant mouse. A chimeric male mouse was mated with the C57BL/6N female mouse, and it gave two male mice that showed transmission to the germ line. The genotype of the Gpbar1 locus was determined through southern blotting analysis, using HindIII-digested genome DNA (Fig. 2B). The band of the wild type had a 11.7 kb length; and the band of the recombinant had a 3.5 kb length.

The disruption of Gpbar1 mRNA expression was analyzed through northern blotting analysis using the poly(A)RNA prepared from the small intestine of a homozygous mouse (Fig. 2C). Before analysis, these mice were back-mated with C57BL/6N mice for four generations. The mice were kept in a cycle of 12 hours light/12 hours dark, and suitably fed with standard rodent solid feed, CA-1 (Clea). A predetermined amount (10 µg) of the poly(A)RNA prepared from the small intestine of three wild-type mice (+/+) and three homozygous mice (-/-) was blotted and hybridized with a [³²P]-labeled probe of mouse Gpbar1 cDNA (Fig. 2C). Gpbar1 had a 1.5 kb length; and β-actin was used as electrophoresis control.

As a result, the homozygous mice gave no the band of Gpbar1, and this confirms the disruption of Gpbar1 mRNA expression in them.

The heterozygous and homozygous mice are livable and reproducible, and were considered normal under a standard laboratory condition with a standard rodent solid feed. Mating of the heterozygous mice produced wild, heterozygous and homozygous mice in a desired Mendelian ratio.

### [Example 3] Analysis of Gpbar1-Deficient Mice for total bile acid pool and fecal bile acid level:

To investigate whether or not Gpbar1 may play a role in maintaining bile acid homeostasis, Gpbar1-deficient mice were analyzed for the total bile acid pool and the fecal bile acid level thereof according to an enzymatic method. All the data are expressed as the mean value ± standard error (SE) of the data of the wild type (+/+), heterozygous (+/-) and homozygous (-/-) mice (n = 7 to 16).

In determination of total bile acid pool and fecal bile acid level, the mice were suitably fed in individual cages. The total bile acid was extracted according to Sinal et al's description (Sinal, C. J., Tohkin, M., Miyata, M., Ward, J. M., Lambert, G., and Gonzalez, F. J.; 2000. Targeted disruption of the nuclear receptor FXR/BAR impairs bile acid and lipid homeostasis, Cell 102: 731-744).

In determination of total bile acid pool, first, the liver, the gallbladder and the entire small intestine were homogenized. With perfusion, a predetermined amount of these tissues was extracted twice with ethanol. Next, in a nitrogen atmosphere, the extract was completely dried and suspended in 50 % ethanol.

In determination of fecal bile acid level, the feces were collected from every mouse for 72 hours just before sacrificed, and these were dried, weighed, and homogenized. Like in the determination of total bile acid pool, a predetermined amount of the sample was extracted.

The total bile acid content of these extracts was determined according to the enzymatic method described by Kitada et al. (Kitada, H., Miyata, M., Nakamura, T., Tozawa, A., Honma, W., Shimada, M., Nagata, K., Sinal, C. J., Guo, G. L., Gonzalez, F. J., and Yamazoe, Y.; 2003, Protective role of hydroxysteroid sulfotransferase in lithocholic acid-induced liver toxicity, J. Biol. Chem., 278: 17838-17844).

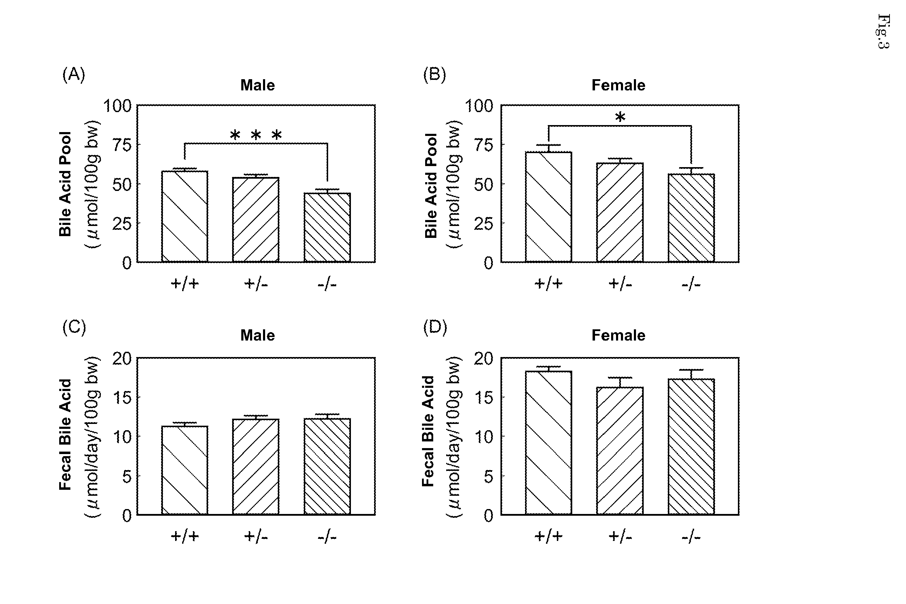

As a result of the above-mentioned determination, it was found that the total bile acid pool of the male and female homozygous mice significantly decreased by 25 % and 21 %, respectively, as compared with that of wild-type mice, as in Fig. 3A and Fig. 3B. To investigate whether or not a nuclear bile acid receptor FXR may have some influence on the phenotype, the total RNA prepared from the liver and the small intestine of the Gpbar1-deficient mouse was analyzed through northern blotting analysis.
The expression level of FXR mRNA was on the same level in the three genotypes (data not shown). From the data, it is suggested that Gpbar1 may not have any influence on the FXR gene expression but may contributes to the regulation of bile acid homeostasis. Though the total bile acid pool decreases, the fecal bile acid level was on the same level in the three genotypes (Figs. 3C, 3D). It is suggested that the bile acid synthesis is not derived for compensating the decrease in bile acid pool (by 21 to 25 %) in homozygous mice.

### [Example 4] Determination of Plasma Triglyceride (TG) and Total Cholesterol:

The plasma triglyceride (TG) and the total cholesterol in homozygous mice and wild-type mice were determined. The plasma triglyceride (TG) and the total cholesterol were measured, using a commercially-available kit [Determiner L-TGII and TC II (Kyowa Medex)].

The plasma triglyceride level of the homozygous mice was the same as that of the wild-type mice. However, the plasma total cholesterol level significantly increased by 16 % in the male homozygous mice (p < 0.05), and its increase was not seen in the female mice (data not shown). From the result, it is suggested that Gpbar1 may have a possibility of its contributing to the regulation of the plasma cholesterol concentration in sexual dimorphic modernity.

### [Example 5] Body Weight Fluctuation of Gpbar1-Deficient Mice fed with ordinary feed:

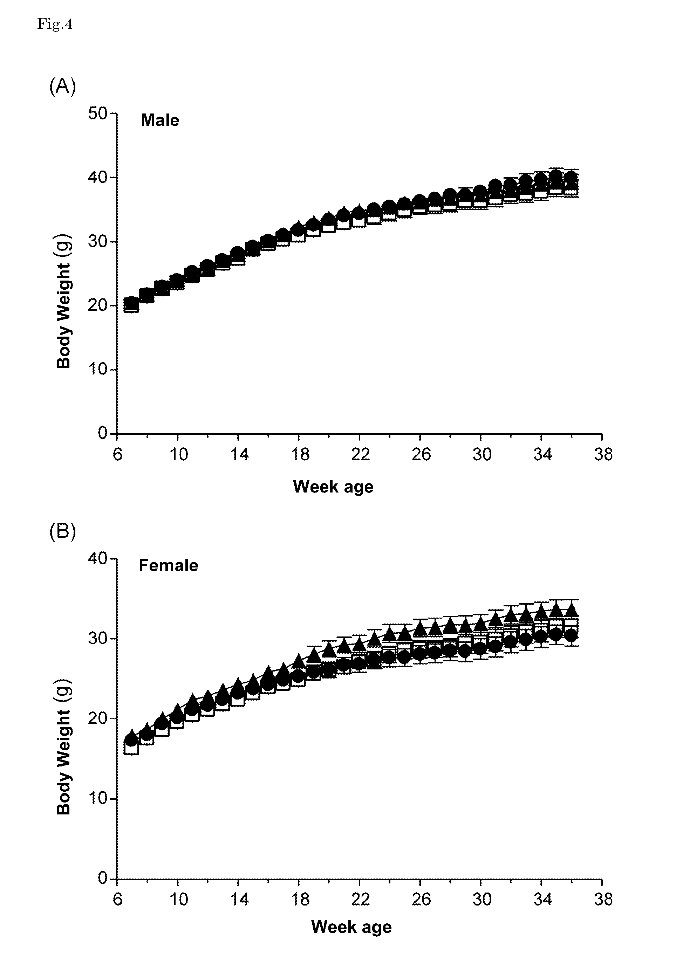

To investigate the influence of Gpbar1 gene deficiency on mice, the time-dependent body weight fluctuation of Gpbar1 homozygous mice and heterozygous mice fed with ordinary feed was recorded (n = 10). As a result, the body weight of male and female homozygous mice did not differ from that of wild-type mice (Fig. 4).

In addition, the total bile acid and lipid concentration in the plasma of these mice was determined. As a result, the plasma total bile acid and triglyceride concentration did not differ between the wild-type mice and the homozygous mice; but the plasma total cholesterol concentration significantly increased in the male mice (16 %, p < 0.05) (data not shown).

### [Example 6] Body Weight Fluctuation and Body Composition Analysis of Gpbar1-Deficient Mice fed with high fat feed:

To investigate the influence of Gpbar1 on fat accumulation, male and female homozygous mice group, heterozygous mice group and wild-type mice group were fed with high fat feed, and the body weight fluctuation of these groups (n = 10) was checked at different times. Through the experiment, the mice of each group were managed in a light-dark cycle of 12 hours light/12 hours dark. From 9-week age to 18-week age, they were freely fed with high-fat feed (60 % calorie lard; by RESEARCH DIETS, New Jersey, USA). The body weight was measured once a week, at 13:00.

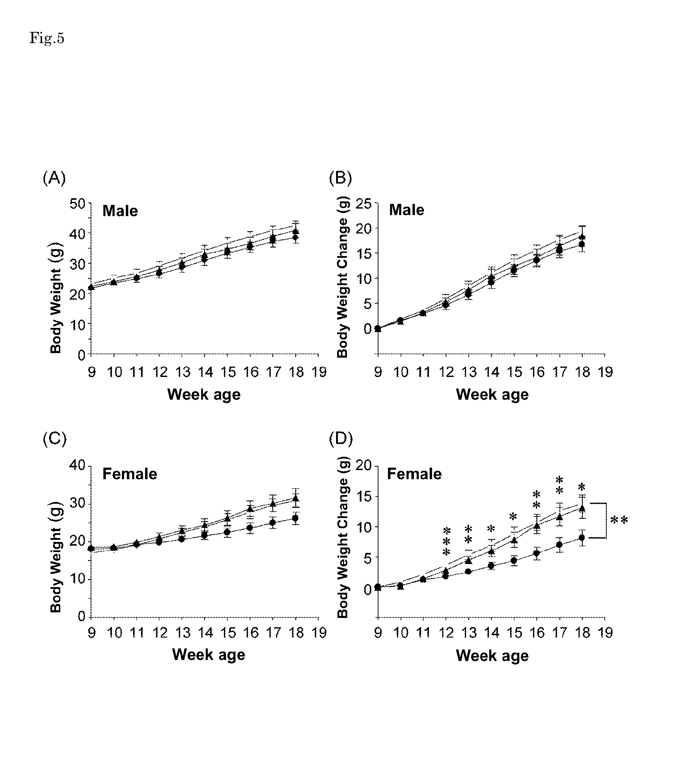

The results are shown in Fig. 5. Figs. 5A and 5C show the body weight of the male and female mice of each group; Figs. 5B and 5D show the body weight change. White squares, black triangles and black rounds indicate homozygous mice group, heterozygous mice group and wild-type mice group, respectively.

As a result, from 12-week age, the body weight of the female homozygous mice group increased as compared with that of the wild-type mice group, and a significant difference was admitted in the body weight change between the two (Figs. 5C, 5D). No significant difference was admitted both in the body weight and in the body weight increase between the female heterozygous mice group and the wild-type mice group, but a significant increase was admitted in the two (Figs. 5C, 5D). On the other hand, the body weight and the body weight increase in the male homozygous mice group and the male heterozygous mice group were seen to increase as compared with those of the wild-type mice group, but it was not remarkable (Figs. 5A, 5B).

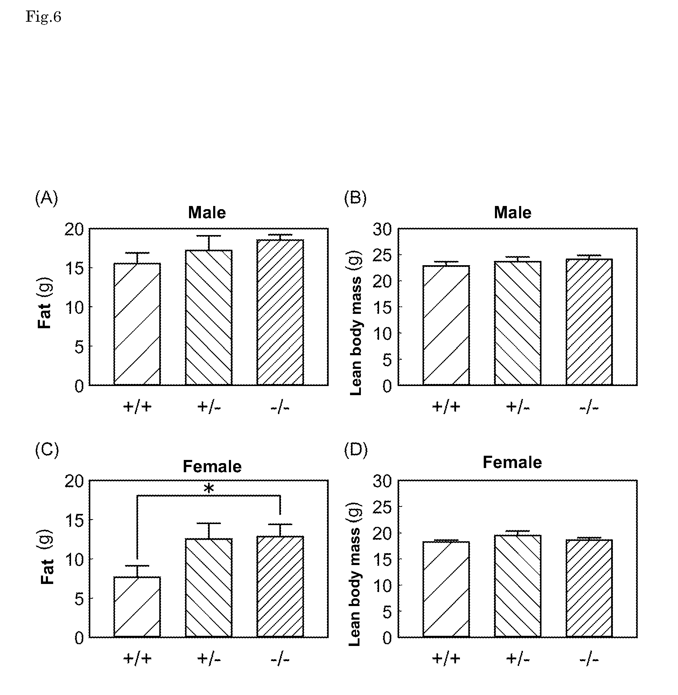

Next, to investigate whether fat accumulation may contribute to the body weight increase in Gpbar1-deficient mice fed with high fat feed, the body composition of the mice of each group was analyzed through nuclear magnetic resonance. The results are shown in Fig. 6. Figs. 6A and 6C show the fat amount of the male and female 18-week age mice of each group; and Figs. 6B and 6D show the fat-excluded body weight thereof. The homozygous mice group, the heterozygous mice group and the wild-type mice group are represented by -/-, +/- and +/+, respectively.

As a result, the fat-excluded body weight of the female homozygous mice group and the female heterozygous mice group was nearly the same as that of the female wild-type mice group (Fig. 6D); but the fat amount of the former was remarkably larger than that of the female wild-type mice group (Fig. 6C). There was admitted a statistical significant difference in the fat amount between the female homozygous mice group and the female wild-type mice group (Fig. 6C). On the other hand, the fat amount in the male homozygous mice group and the male heterozygous mice group increased as compared with that in the wild-type mice group, but it was not remarkable (Fig. 6A). The fat-excluded body weight of the male mice in each group was nearly the same (Fig. 6B).

### INDUSTRIAL APPLICABILITY

The expression level or the activity of Gpbar1 or the bindability to Gpbar1 may be utilized as an index for screening of drugs for treatment or prevention of diseases that accompany changes in total bile acid pool or lipid metabolism disorders, and also for tests for those disorders.
The Gpbar1-deficient mice of the invention may be used as diseased model mice for studies to clarify the physiological role of Gpbar1; and they may be utilized for presuming the side effect of the drugs specifically selected according to the screening method of the invention or that of Gpbar1 inhibitors such as an anti-Gpbar1 antibody or Gpbar1-antagonist low molecules.
Further, the cell line established from the tissue of the genetically-modified animal can be utilized in investigating the side effect of the above-mentioned drugs in an *in-vitro* system.

## Claims

1. A screening method for candidate compounds for a drug for treatment or prevention of diseases that accompany a decrease in total bile acid pool or lipid metabolism disorders, comprising the following steps (a) to (c):
(a) a step of contacting a test compound with Gpbar1,
(b) a step of detecting the binding of the test compound to the Gpbar1,
(c) as step of selecting the test compound that binds to the Gpbar1.

2. A screening method for candidate compounds for a drug for treatment or prevention of diseases that accompany a decrease in total bile acid pool or lipid metabolism disorders, comprising the following steps (a) to (c):
(a) a step of contacting a test compound to a cell that expresses Gpbar1,
(b) a step of determining the expression level of the Gpbar1,
(c) a step of selecting the test compound that increased the expression level of the Gpbar1 as compared with a case not contacted with the test compound.

3. A screening method for candidate compounds for a drug for treatment or prevention of diseases that accompany a decrease in total bile acid pool or lipid metabolism disorders, comprising the following steps (a) to (d):
(a) step of providing a cell or cell extract having a DNA of such that a reporter gene functionally binds to the downstream of the promoter region of a Gpbar1-encoding DNA,
(b) a step of contacting a test compound with the cell or cell extract,
(c) a step of determining the expression level of the reporter gene in the cell or cell extract,
(d) a step of selecting the test compound that increased the expression level of the reporter gene as compared with a case not contacted with the test compound.

4. A screening method for candidate compounds for a drug for treatment or prevention of diseases that accompany a decrease in total bile acid pool or lipid metabolism disorders, comprising the following steps (a) to (c):
(a) a step of contacting a test compound with a cell that has expressed Gpbar1 on the cell surface, in the presence of a ligand to Gpbar1,
(b) a step of determining the activity of Gpbar1 in the cell,
(c) a step of selecting the test compound that increased the activity, as compared with a case not contacted with the test compound.

5. A screening method for candidate compounds for a drug for treatment or prevention of diseases that accompany a decrease in total bile acid pool or lipid metabolism disorders, comprising the following steps (a) to (c):
(a) a step of administering a test compound to a genetically-modified non-human mammal in which the expression of a Gpbar1 gene is artificially inhibited,
(b) a step of determining the total bile acid pool in the genetically-modified non-human mammal,
(c) a step of selecting the compound that increased the total bile acid pool in the genetically-modified non-human mammal, as compared with a case not administered with the test compound.

6. A genetically-modified non-human mammal in which the expression of a Gpbar1 gene is artificially inhibited.

7. A genetically-modified non-human mammal in which an exogenous gene is inserted into one or both of the gene pair of a Gpbar1 gene.

8. The genetically-modified non-human mammal as claimed in claim 6 or 7, which is a model animal for diseases that accompany a decrease in total bile acid pool or lipid metabolism disorders.

9. A genetically-modified mammal cell in which the expression of a Gpbar1 gene is artificially inhibited.

10. A genetically-modified mammal cell in which an exogenous gene is inserted into one or both of the gene pair of a Gpbar1 gene.

11. A drug for treatment or prevention of diseases that accompany a decrease in total bile acid pool or lipid metabolism disorders, which comprises, as the active ingredient thereof, a DNA coding for a Gpbar1 protein.

12. A drug for treatment or prevention of diseases that accompany a decrease in total bile acid pool or lipid metabolism disorders, which is selected according to the screening method of any of claims 1 to 5.

13. A screening method for candidate compounds for a drug for treatment or prevention of diseases that accompany an increase in total bile acid pool or lipid metabolism disorders, comprising the following steps (a) to (c):
(a) a step of contacting a test compound with Gpbar1,
(b) a step of detecting the binding of the test compound to the Gpbar1,
(c) a step of selecting the test compound that binds to the Gpbar1.

14. A screening method for candidate compounds for a drug for treatment or prevention of diseases that accompany an increase in total bile acid pool or lipid metabolism disorders, comprising the following steps (a) to (c):
(a) a step of contacting a test compound to a cell that expresses Gpbar1,
(b) a step of determining the expression level of the Gpbar1,
(c) a step of selecting the test compound that decreased the expression level of the Gpbar1 as compared with a case not contacted with the test compound.

15. A screening method for candidate compounds for a drug for treatment or prevention of diseases that accompany an increase in total bile acid pool or lipid metabolism disorders, comprising the following steps (a) to (d):
(a) step of providing a cell or cell extract having a DNA of such that a reporter gene functionally binds to the downstream of the promoter region of a Gpbar1-encoding DNA,
(b) a step of contacting a test compound with the cell or cell extract,
(c) a step of determining the expression level of the reporter gene in the cell or cell extract,
(d) a step of selecting the test compound that decreased the expression level of the reporter gene as compared with a case not contacted with the test compound.

16. A screening method for candidate compounds for a drug for treatment or prevention of diseases that accompany an increase in total bile acid pool or lipid metabolism disorders, comprising the following steps (a) to (c):
(a) a step of contacting a test compound with a cell that has expressed Gpbar1 on the cell surface,
(b) a step of determining the activity of Gpbar1 in the cell,
(c) a step of selecting the test compound that decreased the activity, as compared with a case not contacted with the test compound.

17. A drug for treatment or prevention of diseases that accompany an increase in total bile acid pool or lipid metabolism disorders, which comprises, as the active ingredient thereof, a compound that lowers the expression or the activity of Gpbar1.

18. A drug for treatment or prevention of diseases that accompany an increase in total bile acid pool or lipid metabolism disorders, which is selected according to the screening method of any of claims 13 to 16.

19. A test method for diseases that accompany a decrease or an increase in total bile acid pool or lipid metabolism disorders, which comprises a step of determining the amount of Gpbar1 gene expression.

20. A test method for diseases that accompany a decrease or an increase in total bile acid pool or lipid metabolism disorders, which comprises a step of detecting the mutation in a Gpbar1 gene region.

21. A test reagent for diseases that accompany a decrease or an increase in total bile acid pool or lipid metabolism disorders, which contains an oligonucleotide capable of hybridizing with a Gpbar1 gene region and having a chain length of at least 15 nucleotides.

22. A test reagent for diseases that accompany a decrease or an increase in total bile acid pool or lipid metabolism disorders, which contains an antibody binding to Gpbar1.
